# EUROPEAN PATENT APPLICATION

(11) **EP 4 656 230 A1**
(43) Date of publication of application: **03.12.2025**
(21) Application number: 24747290.5
(22) Date of filing: 23.01.2024
(51) Int. Cl.: A61M 25/10, A61B 17/56

(54) **MEDICAL BALLOON PRESSURIZER AND MEDICAL BALLOON PRESSURIZER KIT**

(30) Priority: 24.01.2023 JP 2023008547
(71) Applicant: Spine Chronicle Japan Co., Ltd., Kanazawa-city, Ishikawa 920-0848 (JP)
(72) Inventor: YONEZAWA, Noritaka, Kanazawa-city, Ishikawa 9200848 (JP)
(74) Representative: Plougmann Vingtoft a/s
(86) International application number: PCT/JP2024/001808
(87) International publication number: WO 2024/157963

(57) **Abstract**

A medical balloon pressurizer according to the present invention includes a syringe holder, a motion conversion mechanism, a driver, and a support member, and is configured as follows. The syringe holder can be attached to the proximal side of the support member to detachably support an injector equipped with the syringe and the plunger. The motion conversion mechanism can be attached to the distal side of the support member to convert a rotational force of the driver into a force that pushes the plunger of the injector. The driver has a handle which provides a torque limiting function by idling when a torque to rotate the handle exceeds a predetermined value and not transmitting the torque to the motion conversion mechanism.

## Description

### Technical Field

The present invention relates to a medical balloon pressurizer, which is particularly suitable for use in surgeries to dilate a spinal balloon to create a space to be filled with cement in fractured vertebrae and for balloon dilation in blood vessels and in the heart, etc.

### Background Art

Percutaneous vertebroplasty is a treatment for spinal compression fractures in which a needle is inserted into the spine with a compression fracture and medical cement (bone cement, polymethyl methacrylate) is injected through it. Percutaneous vertebroplasty includes percutaneous kyphoplasty (balloon kyphoplasty, BKP), in which a needle is inserted into the spine under general anesthesia, the spine is dilated with a medical balloon (unless otherwise specified, the term "balloon" is used herein to refer to "medical balloon") to create a gap, into which medical cement is injected.

### CITATION LIST

### Patent Document

PTL 1: WO2006/004887

### SUMMARY OF THE INVENTION

### Problems to be Solved by the Invention

In percutaneous kyphosis correction, a surgical instrument with a small medical balloon is inserted into a fractured bone. By gradually dilating this medical balloon, the crushed bone is lifted and returned to its pre-fracture shape as much as possible. When this medical balloon is removed from the affected bone, a space is created there, which is then filled with bone cement. As the bone cement hardens, it is able to support the fractured bone, thus eliminating bone-derived pain during standing and walking.

This medical balloon is injected with a contrast agent to dilate it. A physician dilates the medical balloon while checking the dilation status of the medical balloon injected with the contrast agent on a navigator device. For example, PTL 1 describes a treatment method in which a contrast agent is injected into a balloon inserted into a body using a dilating injector to dilate the balloon.

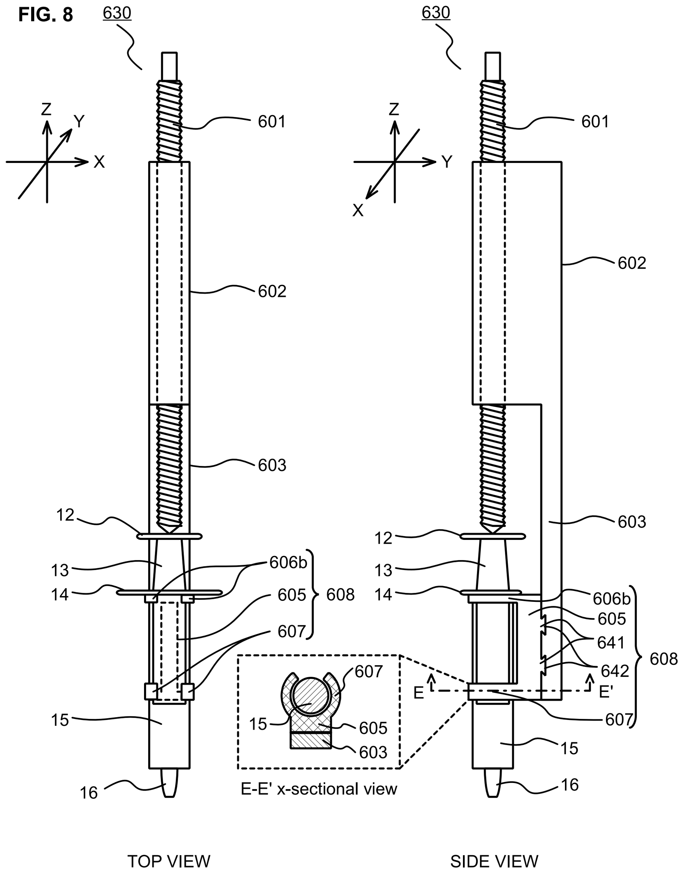

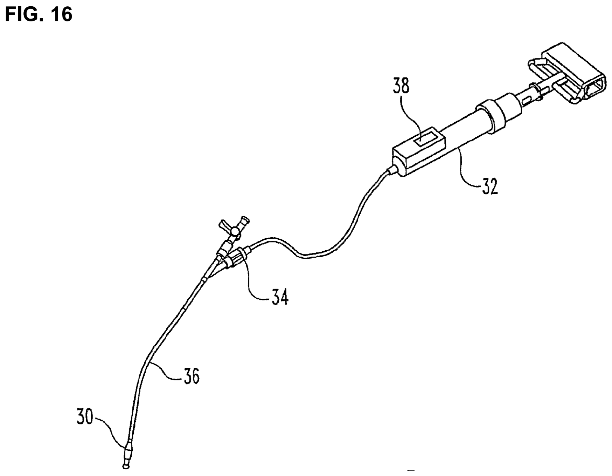

FIG. 16 is an explanatory view illustrating an example configuration of a related art medical balloon dilation system. That is, FIG. 16 corresponds to FIG. 8 of PTL 1. The related art medical balloon dilation system, as described in FIG. 16, uses a specialized balloon indeflator for pressurized injection of a contrast agent into a medical balloon attached to the tip of a catheter. The balloon indeflator pressurizes and extrudes the contrast agent injected into a syringe by rotating and pushing a threaded plunger. The thus extruded contrast agent is injected into the medical balloon.

During injection of the contrast agent into this balloon, the balloon may be ruptured if pressure above a predetermined value is applied to the balloon. To prevent this, the syringe of the indeflator is equipped with a pressure gauge integrated with the syringe to check the pressure on the balloon at all times during pressurization.

Since this indeflator injects and dispenses the contrast agents to be injected into the patient, it has been pharmaceutically approved on the premise that once used, it cannot be reused and must be disposed completely. However, since this indeflator is a very expensive medical device, patients must also bear the cost of disposal of this expensive indeflator in treatment. Therefore, although percutaneous kyphosis correction is a very effective technique, it imposes a heavy financial burden on patients.

An object of the present invention is to reduce the cost of percutaneous kyphosis correction, and therefore reduce the price of items that must be discarded after each surgery.

Means for solving these problems will be described below, but other issues and novel features will become apparent from the description herein and the accompanying drawings.

### Means for Solving Problems

A medical balloon pressurizer according to the present invention includes a syringe holder, a motion conversion mechanism, a driver, and a support member, and is configured as follows.

The syringe holder can be attached to the proximal side of the support member to detachably support an injector equipped with the syringe and the plunger.

The motion conversion mechanism can be attached to the distal side of the support member to convert a rotational force of the driver into a force that pushes the plunger of the injector.

The driver has a handle which provides a torque limiting function by idling when a torque to rotate the handle exceeds a predetermined value and not transmitting the torque to the motion conversion mechanism.

In the present invention, a medical balloon pressurizer consists of a syringe holder, a motion conversion mechanism (which may be composed of a cylinder and a shaft), a support member (called a cylinder support member when the motion conversion mechanism is composed of a cylinder and a shaft) and a driver. A medical balloon pressurizer with an injector attached thereto is called a medical balloon pressurizer kit. For the purpose of illustrating the figures, a medical balloon pressurizer deprived of the driver is used, which is called a medical balloon pressurizer body.

### Effect of the Invention

The effect to be produced by the above-described embodiment will be briefly described below.

Since the medical balloon pressurizer and the injector are separate, only the injector needs to be replaced in a single surgery, and the medical balloon pressurizer does not have to be discarded and is thus reusable. Injectors are inexpensive because general-purpose ones can be used. This greatly reduces the economic burden on patients, since the price of materials to be disposed of after surgery can be less expensive.

### BRIEF DESCRIPTION OF THE DRAWINGS

FIG. 1 is an explanatory view schematically illustrating the entire medical balloon dilation system used to dilate a balloon using a medical balloon pressurizer, which is the present invention.
FIG. 2 is an explanatory view schematically illustrating front, side and cross-sectional structures to show an example of how to secure an injector to a medical balloon pressurizer body according to a first embodiment of the present invention.
FIG. 3 is an explanatory view illustrating a connection structure of a shaft and a plunger flange of a medical balloon pressurizer kit according to the first embodiment of the present invention.
FIG. 4 is an explanatory view illustrating a correlation between the internal pressure of the syringe and the rotational torque of the shaft in the present invention.
FIG. 5 is an explanatory view schematically illustrating front, side and cross-sectional structures to show an example of how a syringe of a medical balloon pressurizer kit is secured according to a second embodiment of the present invention.
FIG. 6 is an explanatory view schematically illustrating front, side and cross-sectional structures to show an example of how a syringe of a medical balloon pressurizer kit is secured according to a third embodiment of the present invention.
FIG. 7 is an explanatory view schematically illustrating front, side and cross-sectional structures to show an example of how a syringe of a medical balloon pressurizer kit is secured according to a fourth embodiment of the present invention.
FIG. 8 is an explanatory view schematically illustrating front, side and cross-sectional structures to show an example of how a syringe of a medical balloon pressurizer kit is secured according to a fifth embodiment of the present invention.
FIG. 9 is an explanatory view schematically illustrating front, side and cross-sectional structures to show an example of how a syringe of a medical balloon pressurizer kit is secured according to a sixth embodiment of the present invention.
FIG. 10 is an explanatory view schematically illustrating front, side and cross-sectional structures to show an example of how a syringe of a medical balloon pressurizer kit is secured according to a seventh embodiment of the present invention.
FIG. 11 is an explanatory view illustrating an example rotation of a syringe holder of the medical balloon pressurizer kit according to the seventh embodiment of the present invention.
FIG. 12 is an explanatory view schematically illustrating front, side and cross-sectional structures to show an example of how a syringe of a medical balloon pressurizer kit is secured according to an eighth embodiment of the present invention.
FIG. 13 is an explanatory view schematically illustrating a structure to show an example of how a syringe of a medical balloon pressurizer kit is secured according to a ninth embodiment of the present invention.
FIG. 14 is an explanatory view schematically illustrating a structure to show another example of how a syringe of a medical balloon pressurizer kit is secured according to a 10th embodiment of the present invention.
FIG. 15 is an explanatory view illustrating an example configuration connecting a shaft and the plunger flange of a medical balloon pressurizer kit of the present invention.
FIG. 16 is an explanatory view illustrating an example configuration of a related art medical balloon dilation system.
FIG. 17 is an explanatory view schematically illustrating the entire medical balloon dilation system used to dilate a balloon using a medical balloon pressurizer according to a 12th embodiment of the present invention.
FIG. 18 is an explanatory view schematically illustrating an example configuration that allows a syringe holder to be attached to and detached from a medical balloon pressurizer body according to a 13th embodiment of the present invention.
FIG. 19 is an explanatory view schematically illustrating an example configuration that allows an injector to be attached to and detached from a medical balloon pressurizer body according to a 14th embodiment of the present invention.
FIG. 20 is an explanatory view schematically illustrating an example configuration that allows an injector to be attached to and detached from the medical balloon pressurizer body according to a 15th embodiment of the present invention.
FIG. 21 is an explanatory view schematically illustrating an example configuration that allows an injector to be attached to and detached from the medical balloon pressurizer body according to a 16th embodiment of the present invention.
FIG. 22 is an explanatory view schematically illustrating an example configuration for securing an injector to be attached to and detached from the medical balloon pressurizer body according to a 17th embodiment of the present invention.
FIG. 23 is an explanatory view schematically illustrating an example of a rapid depressurization mechanism in a medical balloon pressurizer body according to an 18th embodiment of the present invention.
FIG. 24 is an explanatory view schematically illustrating a first example of a rapid depressurization mechanism (coil spring) in the medical balloon pressurizer body according to the 18th embodiment of the present invention.
FIG. 25 is an explanatory view schematically illustrating a second example of a rapid depressurization mechanism (plate spring) in the medical balloon pressurizer body according to the 18th embodiment of the present invention.
FIG. 26 is an explanatory view schematically illustrating a third example of a rapid depressurization mechanism (modification of the coil spring) in the medical balloon pressurizer body according to the 18th embodiment of the present invention.
FIG. 27 is an explanatory view schematically illustrating an example of a rapid depressurization mechanism (before the plunger is pushed in) in a medical balloon pressurizer body according to a 19th embodiment of the present invention.
FIG. 28 is an explanatory view schematically illustrating an example of a rapid depressurization mechanism (after the plunger is pushed in) in the medical balloon pressurizer body according to the 19th embodiment of the present invention.
FIG. 29 is an explanatory view schematically illustrating an example of a rapid depressurization mechanism (screw engagement release) in the medical balloon pressurizer body according to the 19th embodiment of the present invention.
FIG. 30 is an explanatory view schematically illustrating an example of a rapid depressurization mechanism (plunger withdrawal) in the medical balloon pressurizer body according to the 19th embodiment of the present invention.
FIG. 31 is an explanatory view schematically illustrating an example of a rapid depressurization mechanism (before a plunger is pushed in) in a medical balloon pressurizer body according to a 20th embodiment of the present invention.
FIG. 32 is an explanatory view schematically illustrating an example of a rapid depressurization mechanism (after a plunger is pushed in) in the medical balloon pressurizer body according to the 20th embodiment of the present invention.
FIG. 33 is an explanatory view schematically illustrating an example of a rapid depressurization mechanism (screw engagement release) in a medical balloon pressurizer body according to the 20th embodiment of the present invention.
FIG. 34 is an explanatory view schematically illustrating an example of a rapid depressurization mechanism (plunger withdrawal) in the medical balloon pressurizer body according to the 20th embodiment of the present invention.

### DETAILED DESCRIPTION OF THE INVENTION

### 1. Outline of Embodiment

First, an outline of a representative embodiment disclosed in the present application will be given. Reference codes in the drawings that are referred to in parentheses in the outline description of representative embodiment are merely illustrative of what is included in the concept of the components to which they refer.

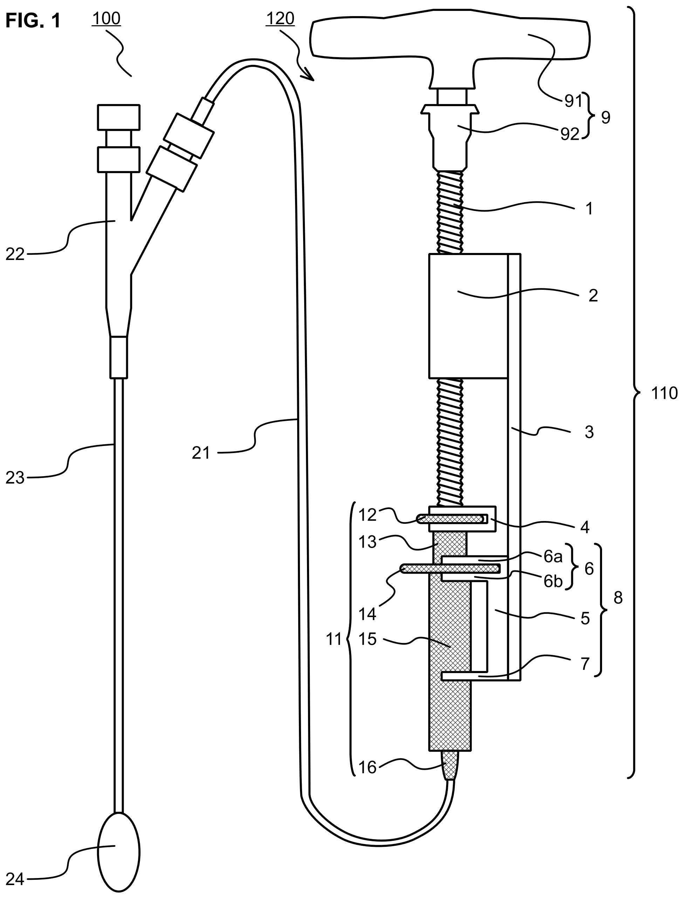

### [1] Medical balloon pressurizer equipped with detachable injector and protected by torque limiting mechanism (FIGs. 1 to 3, 5 to 15, 17 to 30)

A representative embodiment disclosed in this application is a medical balloon pressurizer (120) including a syringe holder (8), a motion conversion mechanism ({1, 2}, {31, 32, 33}, {2, 41, 42}), a driver (9) and a support member (3), and is configured as follows.

The syringe holder can be attached to the proximal side of the support member to detachably support an injector (11) equipped with a syringe (15) and a plunger (13).

The motion conversion mechanism can be attached to the distal side of the support member to convert a rotational force of the driver into a force that pushes the plunger (13) of the injector.

The driver has a handle (91) which provides a torque limiting function by idling when a torque to rotate the handle exceeds a predetermined value and not transmitting the torque to the motion conversion mechanism.

Since the medical balloon pressurizer and the injector are separate, only the injector needs to be replaced in a single surgery, and the medical balloon pressurizer does not need to be discarded and is thus reusable. Injectors are inexpensive because general-purpose ones can be used. This greatly reduces the economic burden on patients, since the price of materials to be disposed of after surgery can be less expensive.

### [2] Syringe holder and support member are integrated (FIGs. 1, 2, 5, 6, 12, 20, 21, 22)

The medical balloon pressurizer of [1] is configured as follows.

The injector includes a finger flange (14) provided at a distal end of the syringe and a plunger flange (12) provided at a distal end of the plunger.

The motion conversion mechanism has a rotary motion section ({1, 2}, {32, 33}, {42, 2}) that is connected to the driver and to which the torque is applied, and a linear motion section (1, 31, 41) that contacts the plunger flange and pushes the plunger.

The syringe holder has a syringe fixing section (7) that detachably supports the syringe of the injector and a syringe-side finger flange fixing section (6b) that contacts and supports the proximal side of the finger flange of the injector.

This provides a practical, simple embodiment of the medical balloon pressurizer according to the present invention.

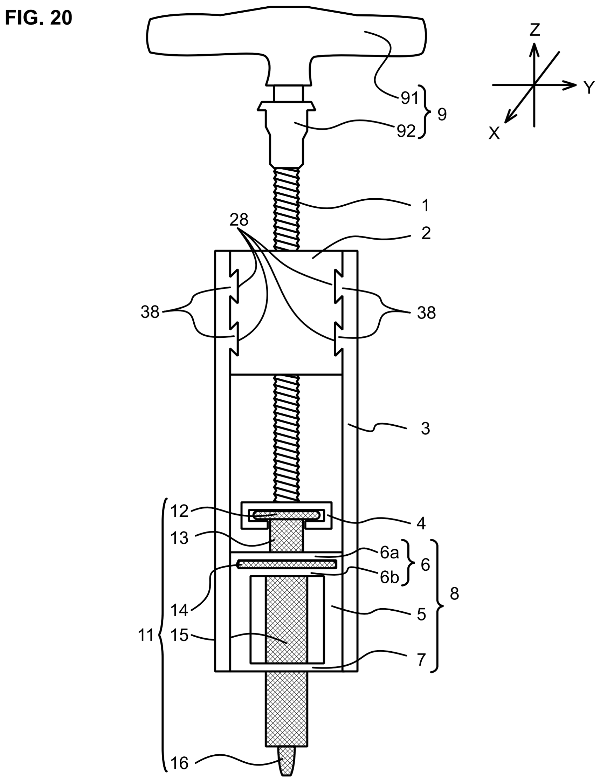

### [3] Double-sided support member (FIGs. 20-22)

In the medical balloon pressurizer of [2], the support member (3) is formed as two beams, so that the syringe holder and the motion conversion mechanism (composed of the shaft 1 and the cylinder 2) are each supported by the two beam-shaped support members.

This ensures that the syringe holder (8) and the motion conversion mechanism (composed of the shaft 1 and the cylinder 2) are firmly aligned on the same axis and prevents twisting and other problems associated with the rotation of the driver (9).

### [4] Detachable motion conversion mechanism (FIG. 20)

In the medical balloon pressurizer of [3], the motion conversion mechanism is detachably attached by being inserted and removed along a slide mechanism (28, 38) provided in the two beam-shaped support members.

This facilitates the attachment and removal of the injector to and from the syringe holder. This is because the injector can be attached by inserting the injector into the syringe holder from the direction in which the motion conversion mechanism should be attached while the motion conversion mechanism is removed, and then the motion conversion mechanism can be attached.

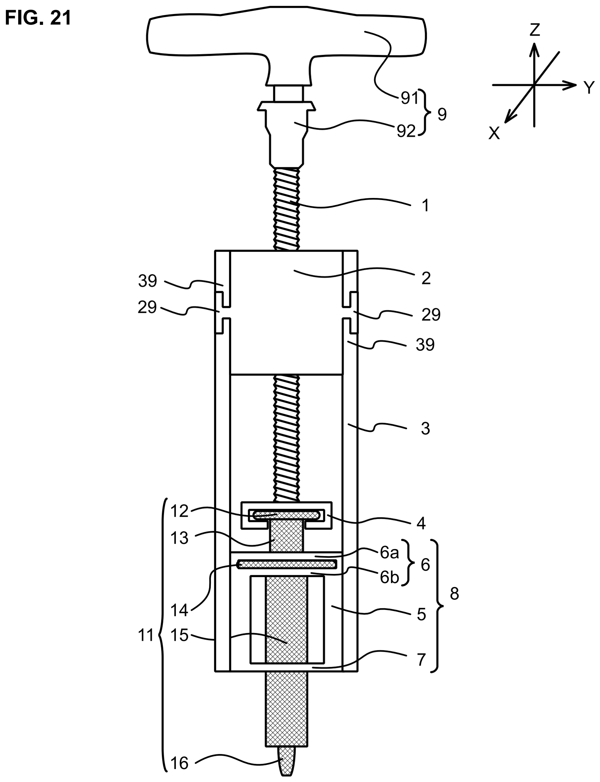

### [5] Motion conversion mechanism which rotates (FIG. 21)

In the medical balloon pressurizer of [3], the motion conversion mechanism is mounted, by a rotating mechanism (29, 39) on the two beam-shaped support members, to be oriented in a direction in which the syringe holder is attached and another direction.

This facilitates the attachment and removal of the injector to and from the syringe holder. This is because, by directing the pusher extending from the motion conversion mechanism and pushing the plunger flange of the injector, to a direction different from that of the syringe holder, a space is created above the syringe holder, so the injector is inserted through that space into the syringe holder and attached; then the direction of the pusher is returned to its original position so that a member that pushes the plunger flange can be attached to the plunger flange of the injector attached to the syringe holder.

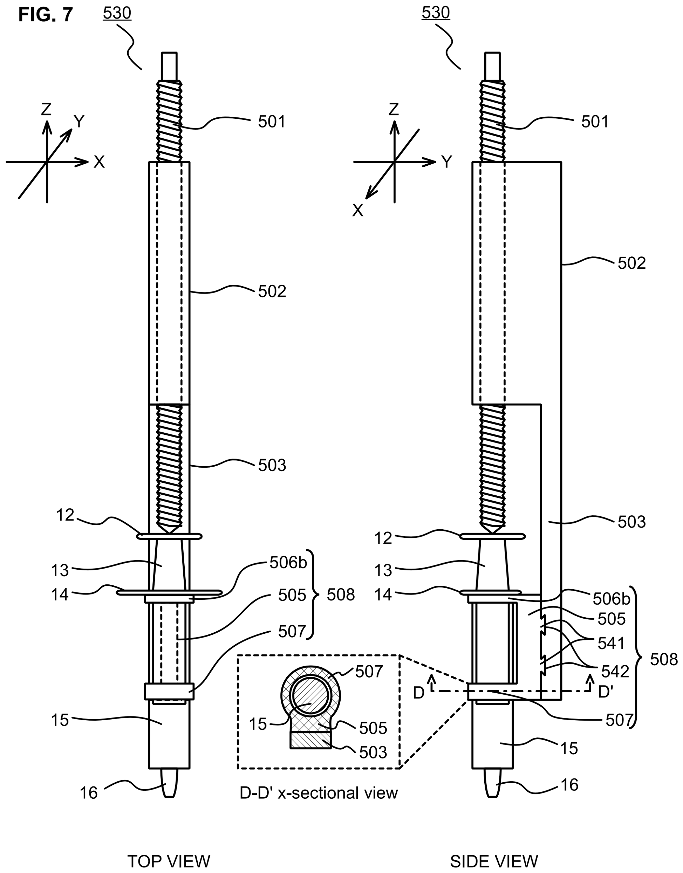

### [6] Syringe holders can be attached to and detached from support member (FIGs. 7, 8, 9, 13, 14, 18)

In the medical balloon pressurizer of [1], the syringe holder is detachably attached to the support member.

This facilitates the attachment and removal of the injector to and from the syringe holder. This is because the syringe holder can be removed from the support member, the injector can be attached to the syringe holder, and then the syringe holder with the injector attached can be attached to the support member.

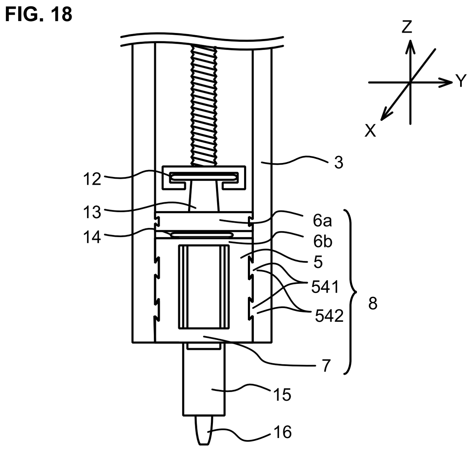

### [7] Double-sided support member (FIG. 18)

In the medical balloon pressurizer of [6], the support member is formed as two beams, so that the syringe holder and the motion conversion mechanism are each supported by the two beam-shaped support members.

This ensures that the syringe holder and the motion conversion mechanism are firmly aligned on the same axis, suppressing twisting and other effects caused by the rotation of the driver.

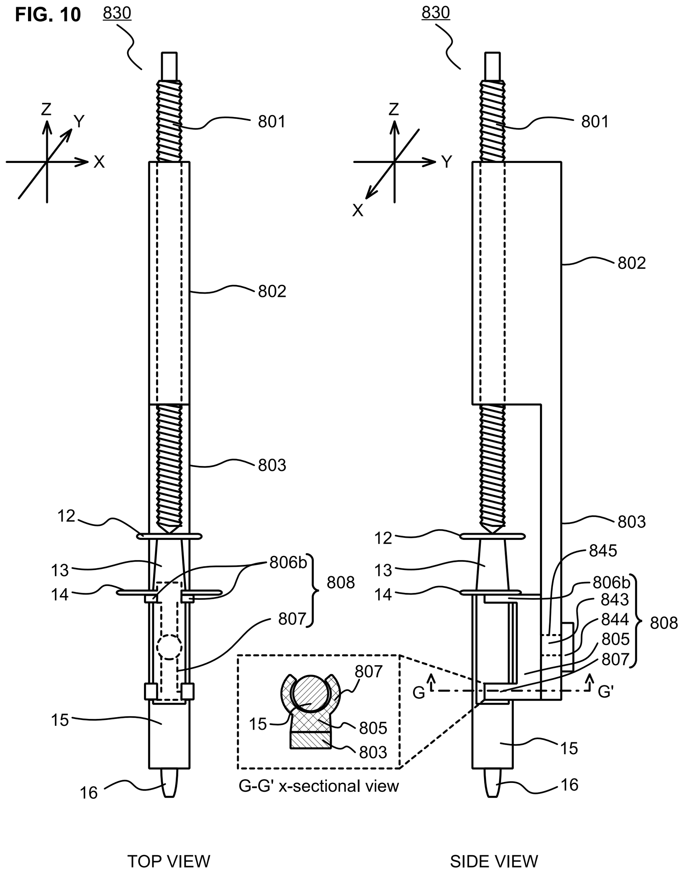

### [8] Syringe holder can rotate on support member (FIGs. 10, 11, 19).

In the medical balloon pressurizer of [1], the syringe holder is mounted, by a rotating mechanism (843 to 845) on the two beam-shaped support members, to be oriented in a direction in which the motion conversion mechanism is attached and another direction.

This facilitates the attachment and removal of the injector to and from the syringe holder. This is because, by rotating the syringe holder and directing a member that pushes the plunger flange of the injector, which extends from the motion conversion mechanism, to a direction different from that of the syringe holder, a space is created above the syringe holder, so the injector is inserted through that space into the syringe holder and attached; then the direction of the pusher is returned to its original position so that a member that pushes the plunger flange can be attached to the plunger flange of the injector attached to the syringe holder.

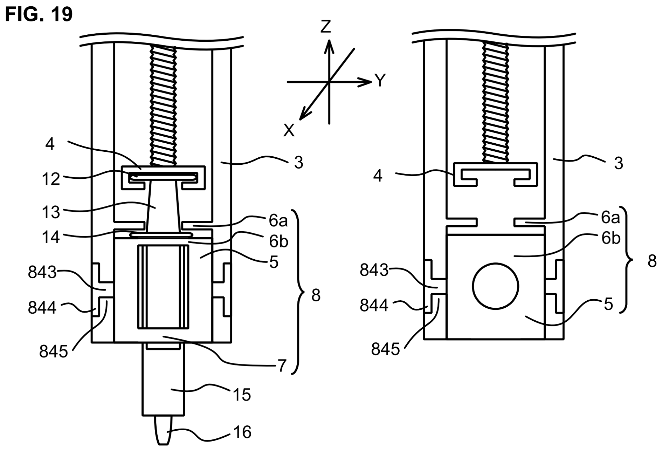

### [9] Double-sided support member (FIG. 19)

In the medical balloon pressurizer of [8], the support member is formed as two beams, so that the syringe holder and the motion conversion mechanism are each supported by the two beam-shaped support members.

This ensures that the syringe holder and the motion conversion mechanism are firmly aligned on the same axis, suppressing twisting and other effects caused by the rotation of the driver.

### [10] Motion conversion mechanism consists of cylinder and shaft (FIG. 1).

The medical balloon pressurizer of [1] is configured as follows.

The motion conversion mechanism includes a cylinder (2) which has a female thread formed on its inner surface and a shaft (1) which has a male thread formed that engages with the female thread on the inner surface of the cylinder and passes through the cylinder,

The driver (9) is mounted so that the shaft can be rotated by the handle, the shaft rotates with the rotation of the handle and moves back and forth in the axial direction of the shaft through engagement with the female thread on the inner surface of the cylinder, and is able to move the plunger back and forth in the axial direction of the shaft via a plunger flange provided at a distal end of the plunger, which contacts the shaft at its tip.

This provides a practical, simple embodiment of the medical balloon pressurizer according to the present invention.

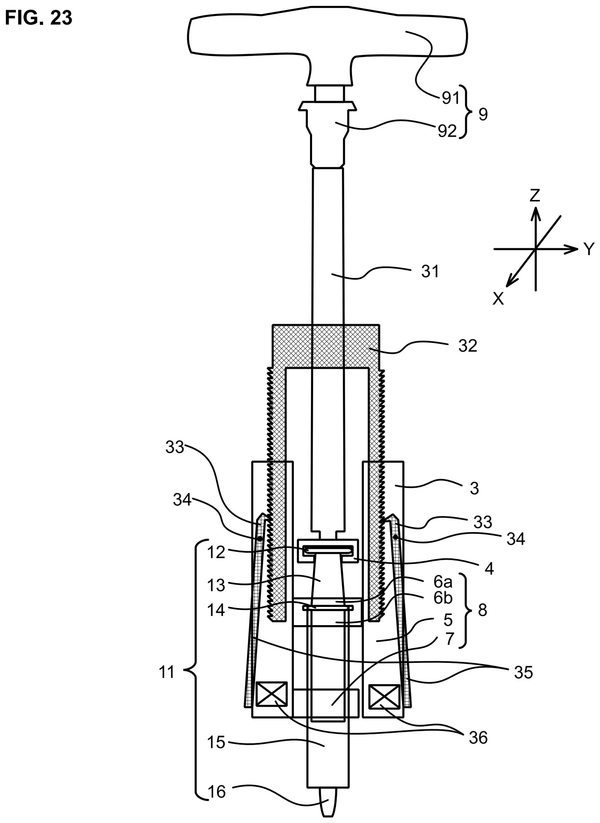

### [11] Rapid depressurization mechanism (FIGs. 23 to 30)

In the medical balloon pressurizer of [1], the motion conversion mechanism has a pusher (31, 41) which is provided along an axis of rotation of the driver and is connected to the plunger flange of the injector, and is configured as follows.

That is, the motion conversion mechanism can switch between a normal state and a rapidly depressurized state with the rotation of the driver, the normal state being a state in which the pushing and pulling of the plunger into and out of the syringe of the injector is controlled by the change of distance between the syringe and the pusher fixed to the syringe holder, and the rapidly depressurized state being a state in which the plunger of the injector can be withdrawn regardless of the rotation of the driver.

This allows for rapid depressurization and deflation of the balloon in unforeseen circumstances.

### [12] Rapid depressurization mechanism using ratchet mechanism inside syringe holder (FIGs. 23 to 26)

In the medical balloon pressurizer of [11], the motion conversion mechanism includes a cylinder (32) that has threads on its outer peripheral surface and rotates with the driver, with the pusher connected on the central axis of rotation, and a ratchet structure (33) that has threads that engage with the threads of the cylinder from the outside of the outer peripheral surface and is fixed to the syringe holder.

The motion conversion mechanism is placed in the normal state by engaging the threads of the cylinder with the threads of the ratchet structure, and is placed in the rapidly depressurized state by a ratchet release by moving the threads of the ratchet structure outward from the outer peripheral surface of the cylinder to disengage them from the threads of the cylinder.

This allows the implementation of an easy-to-operate rapid depressurization mechanism in the motion conversion mechanism. The ratchet mechanism can be operated on the syringe holder side.

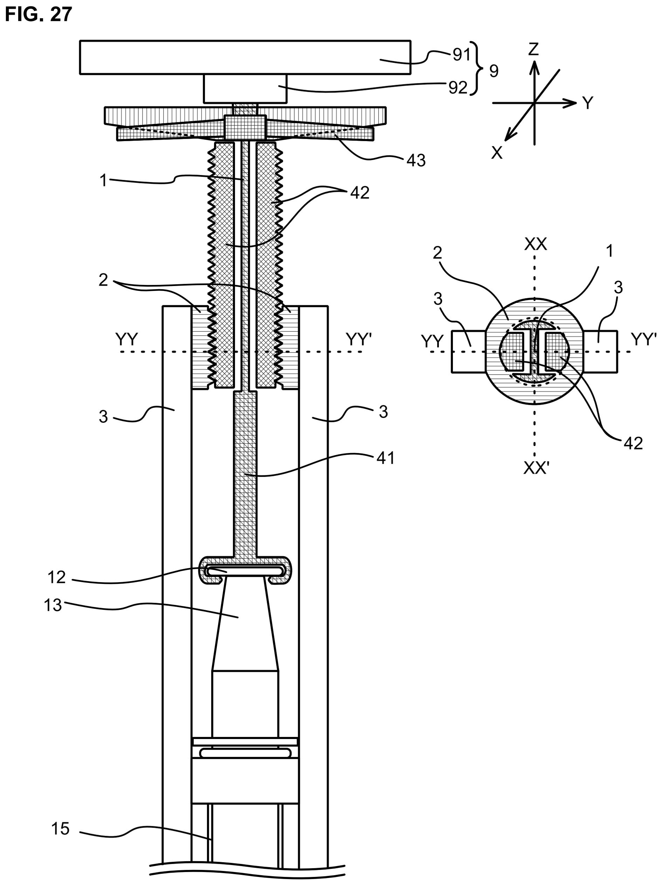

### [13] Rapid depressurization mechanism by controlling engagement with threads of cylinder from shaft side (FIGs. 27 to 30)

In the medical balloon pressurizer of [11], the motion conversion mechanism includes a cylinder (2) that is fixed to the support member and has threads on its inner peripheral surface, a shaft (1) that is inserted inside the cylinder and rotates with the driver, with the pusher connected on the central axis of rotation, and one or more rods (42) that have threads that engage with the threads of the cylinder.

The shaft has grooves in a plane perpendicular to the central axis, each of the one or two or more rods placed in the normal state when it protrudes from the groove and the threads of rod engages with the threads of the cylinder, and placed in the rapidly depressurized state when the rod is accommodated in the groove in the direction of the central axis and disengages the threads of the rod from the threads of the cylinder.

This allows the implementation of an easy-to-operate rapid depressurization mechanism in the motion conversion mechanism. The operation of the rod, i.e., switching between the normal state and the rapidly depressurized state, can be performed on the driver side.

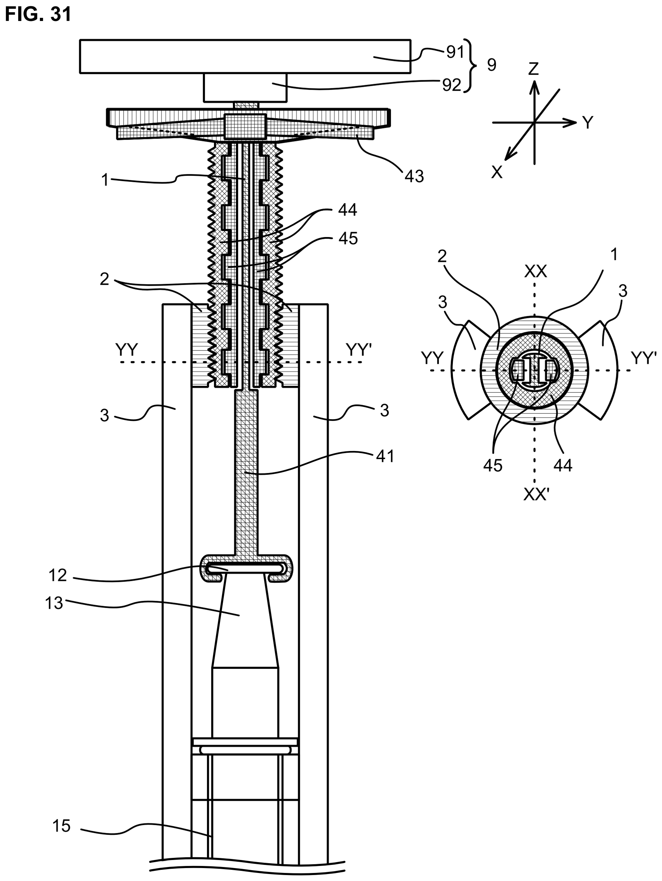

### [14] Rapid depressurization mechanism by controlling presence or absence of connection between inner cylinder and shaft, which engages with threads of cylinder from shaft side (FIGs. 31 to 34)

In the medical balloon pressurizer of [11], the motion conversion mechanism includes a cylinder (2) that is fixed to the support member and has threads on its inner peripheral surface, a shaft (1) that is inserted inside the cylinder and rotates with the driver, with the pusher connected on the central axis of rotation, an inner cylinder with threads that engage with the threads of the cylinder, and a rotation transmission rod (45).

The shaft has grooves in a plane perpendicular to the central axis, the rotary transmission rod is placed in the normal state when a protrusion protruding from the groove and formed on the rotation transmission rod engages with a recess formed on the inner circumference of the inner cylinder, and placed in the rapidly depressurized state when the engagement between the protrusion and the recess is released when the rotary transmission rod is accommodated in the groove in the direction of the central axis.

This allows the implementation of an easy-to-operate rapid depressurization mechanism in the motion conversion mechanism. The operation of the rotation transmission rod, i.e., switching between the normal state and the rapidly depressurized state, can be performed on the driver side.

### [15] Torque limiter setpoints (FIG. 1 to FIG. 4)

In the medical balloon pressurizer of [1], the predetermined value is 2 Nm.

This reduces pressurization so that no pressure is applied that would cause the balloon to rupture. Since there is no need to use conventional equipment to measure the internal pressure of the syringe, the overall cost of the medical balloon dilation system can be reduced.

### [16] Invention of medical balloon pressurizer kit (FIG. 1 to FIG. 4, FIG. 17)

In the medical balloon pressurizer of [1], it is a medical balloon pressurizer kit (110) in which the injector is detachably attached to the syringe holder.

This allows the medical balloon to be dilated. The present invention is an invention of a medical balloon pressurizer kit, which consists of a medical balloon pressurizer with an injector attached.

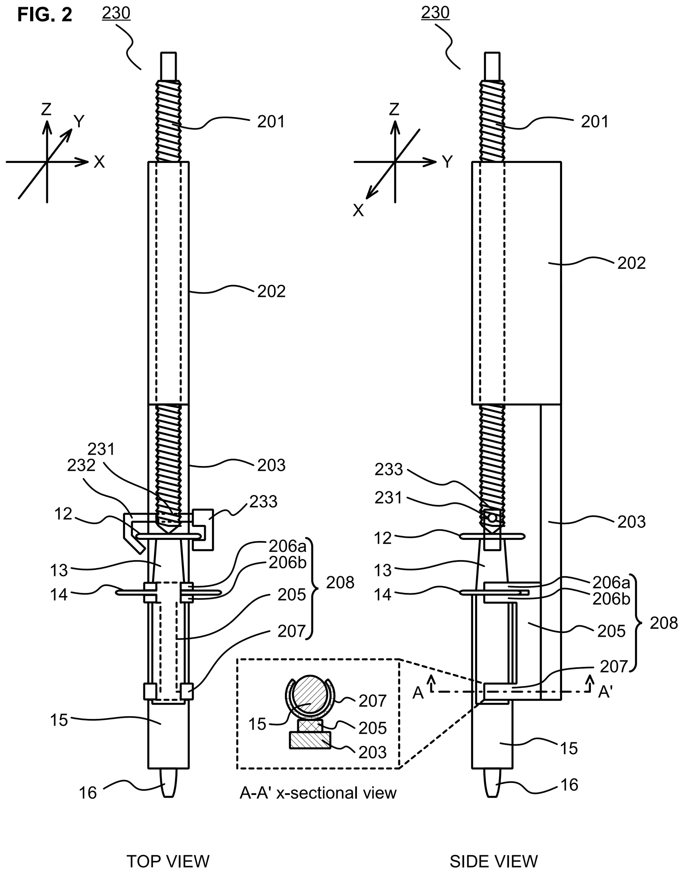

### Medical pressurizer with detachable injector (FIG. 1 and FIG. 2)

A representative embodiment disclosed in this application is a medical balloon pressurizer (120) including a cylinder support member (3) which supports a syringe holder (8) capable of fixing a syringe (15) of an injector (11) and a cylinder (2) having a female thread formed on its inner surface, a shaft (1) with a male thread formed to engage with the female thread on the inner surface of the cylinder and passing through the cylinder, and a driver (9) mounted so that a handle (91) is rotated by the shaft, and is configured as follows.

The syringe holder can detachably secure the syringe of the injector by means of the syringe fixing section (7) and a syringe-side finger flange fixing section (6b).

The shaft rotates with the rotation of the handle and moves back and forth in the axial direction of the shaft through engagement with the female thread on the inner surface of the cylinder, and is able to move the plunger (13) of the injector back and forth in the axial direction of the shaft via a plunger flange, which contacts the shaft at its tip.

The driver has a torque limiting function for idling and not rotating the shaft when the torque to rotate the handle exceeds a predetermined value.

Here, the term "back and forth" means the proximal and distal sides when the above axial direction is perpendicular to the trunk, and is a common expression throughout this specification unless otherwise specified. The axis of the shaft may also be abbreviated as a shaft axis.

This allows the medical balloon pressurizer and the injector to be configured as separate units, so only the injector needs to be replaced in a single surgery. The medical balloon pressurizers do not need to be discarded and can be reused. The injector can be a general-purpose one (a pressure-resistant syringe), so its price is low. This greatly reduces the economic burden on the patient, since the price of the material to be disposed of after surgery can be low.

The configuration and technical features shown in [101] are common to all of the first through ninth embodiments shown below. What is included in the concept of components with that reference codes in FIG. 1 or FIG. 2, which are referred to in parentheses, is illustrated, and reference codes attached to corresponding components in other embodiments are included and illustrated. In each of the following sections, the reference codes of the figures referred to in the embodiment corresponding to that section will be added.

### Means of securing syringe and medical balloon pressurizer (FIG. 1 to FIG. 5)

In the medical balloon pressurizer (120) of [101], the syringe fixing section and the syringe-side finger flange fixing section are ring-shaped to allow the injector to be fixed therethrough.

This allows the syringe to be detachably secured to the medical balloon pressurizer while still being stable and not easily detached from the syringe holder on impact or by other factors.

### Means of securing syringe and medical balloon pressurizer (FIG. 1 to FIG. 4, FIG. 6)

In the medical balloon pressurizer (120) of [101], the syringe fixing section (407) and the syringe-side finger flange fixing section (406b) can detachably fix the injector by extending in an arc shape from both sides of the syringe holder around the side of the syringe of the injector, both ends thereof having a gap through which a scale on the syringe of the injector can be seen when the injector is attached.

This allows the person operating the medical balloon pressurizer kit to see the scale on the syringe even when the syringe is attached to the medical balloon pressurizer. This allows the person operating the medical balloon pressurizer kit to easily monitor the amount of contrast agent held in the syringe.

### Means of securing syringe and medical balloon pressurizer (FIG. 1 to FIG. 4, FIG. 6)

In the medical balloon pressurizer (120) of [103], a shaft-side finger flange fixing section (6a) is provided on the syringe holder (5, 405) that can sandwich the finger flange of the injector to detachably secure together with the syringe-side finger flange fixing section.

The syringe fixing section (7, 407) and the syringe-side finger flange fixing section (6b, 406b) are formed by elastic materials and can deform so that when the injector is pressed against it, the gap widens to the outer diameter of the injector, thereby fixing the injector when it is mounted.

In this manner, to attach the injector to the medical balloon pressurizer, the syringe can be attached to the medical balloon pressurizer by pressing the syringe against the shaft-side finger flange fixing section and the syringe fixing section from a direction parallel to the shaft and perpendicular to the shaft axis direction to push their gaps open. To remove the injector from the medical balloon pressurizer, when the syringe is pulled away from the syringe holder, the gap between the shaft-side finger flange fixing section and the syringe fixing section widens, and the syringe can be removed from the syringe holder. Thus, the injector can be easily attached and detached.

### Means of securing syringe and medical balloon pressurizer (FIG. 1 to FIG. 4, FIG. 7 to FIG. 12, FIG. 14, 15)

In the medical balloon pressurizer (120) of [101], the syringe holder and the cylinder support member are independent members.

This allows the injector to be attached to and removed from the medical balloon pressurizer after the syringe of the injector is secured to the syringe holder, which can then be attached to a cylinder member. In this manner, the injector can easily be detachably secured to the medical balloon pressurizer.

### Means of securing syringe and medical balloon pressurizer (FIG. 1 to FIG. 4, FIG. 7, FIG. 8)

In the medical balloon pressurizer (120) of [105], the cylinder support member has slide grooves (542, 642) extending at right angles to the shaft axis. The cross-section of the slide grooves is narrower at the opening than at the bottom.

The cylinder holder has syringe holder protrusions (541, 641) on the side opposite to the surface where the syringe is to be fixed; syringe holder protrusions fit into the slide grooves to detachably secure to the cylinder support member.

This allows the syringe holder to be easily detached from the cylinder support member.

### Means of securing syringe and medical balloon pressurizer (FIG. 1 to FIG. 4, FIG. 7)

In the medical balloon pressurizer (120) of [106], the syringe fixing section (507) is ring-shaped to allow the injector to be fixed therethrough.

This allows the syringe holder to be easily attached to and detached from the cylinder support member. Furthermore, this allows the syringe to be detachably secured to the medical balloon pressurizer while still being stable and not easily detached from the syringe holder on impact or by other factors.

### Means of securing syringe and medical balloon pressurizer (FIG. 1 to FIG. 4, FIG. 8)

In the medical balloon pressurizer (120) of [106], the syringe fixing section (607) extends in an arc shape that can detachably fix the side surface of the syringe of the injector from both sides of the syringe holder, both ends thereof having a gap through which a scale on the syringe of the injector can be seen when the injector is attached.

This allows the syringe holder to be easily attached to and removed from the cylinder support member. Furthermore, the person operating the medical balloon pressurizer kit can see the scale on the syringe even when the syringe is attached to the medical balloon pressurizer. This allows the person operating the medical balloon pressurizer kit to easily monitor the amount of contrast agent held in the syringe.

### Means of securing syringe and medical balloon pressurizer (FIG. 1 to FIG. 4, FIG. 9)

In the medical balloon pressurizer (120) of [106], the cylinder support member is further provided with a shaft-side finger flange fixing section (706a) that holds and fixes the finger flange of the injector from the plunger flange side.

The shaft side finger flange is arc-shaped extending from the cylinder support member.

This allows the cylinder holder to be easily attached to and removed from the cylinder support member while further securing the attached cylinder so that it does not move in the shaft axis direction.

### Means of securing syringe and medical balloon pressurizer (FIG. 1 to FIG. 4, FIG. 10 to FIG. 11)

In the medical balloon pressurizer (120) of [105], the cylinder holder has a syringe holder protrusion (843) on the opposite side of the syringe holder from the side where the syringe of the injector is fixed.

The cylinder support member has a recess or a through hole (845) that secures the cylinder holder in a rotatable manner in a plane that is parallel to the cylinder support member.

This allows the syringe holder to rotate with respect to the cylinder support member, so when the syringe is mounted on the syringe holder, the syringe holder can be tilted to a position where the syringe and shaft do not collide with each other, and the syringe can be inserted inside the syringe-side finger flange fixing section and the syringe fixing section. Next, after rotating the syringe holder to a position parallel to the shaft axis, the plunger flange is secured to the shaft. In this manner, in the present embodiment, the injector can be easily attached to and removed from the syringe holder.

### Means of securing syringe and medical balloon pressurizer (FIG. 1 to FIG. 4, FIG. 12)

In the medical balloon pressurizer (120) of [101], the syringe fixing section is a ring shape formed at the end (proximal end) of the cylinder support member extending away from the cylinder support member (further proximal direction).

The syringe-side finger flange fixing section (906b) is formed by an elastic material, and the syringe side finger flange fixing section is further provided to extend in an arc shape from both sides of the cylinder support member capable of holding the side surface of the syringe of the injector from both sides of the syringe holder; the gap is widened when the injector is pressed against it, allowing the syringe of the injector to pass through, and the injector can be detachably secured when the injector is attached.

The shaft-side finger flange fixing section extends from both sides of the cylinder support member and is formed so that it can be detachably fixed between the finger flanges together with the syringe-side finger flange fixing section.

This allows the syringe to be moved from diagonally above the front of the medical balloon pressurizer downward to insert the syringe into the syringe fixing section and remove it in the opposite direction. In this manner, the injector can be easily attached to and detached from the medical balloon pressurizer.

### Means of securing syringe and medical balloon pressurizer (FIG. 1 to FIG. 4, FIG. 13, FIG. 14)

In the medical balloon pressurizer (120) of [105], the cylinder support member has a slide groove (1042) extending from its proximal end in the axial direction of the shaft, and a through hole (1045) at a position to restrict movement of the syringe holder toward the tip (proximal end). The cross-section of the slide groove is narrower at the opening than at the bottom.

The syringe holder has a shaft-side finger flange fixing section (1006a) at a position where the finger flange can be detachably fixed together with the syringe-side finger flange fixing section, and the syringe holder protrusion (1041) on the side opposite to the surface where the syringe of the injector is to be fixed so that the syringe holder protrusion fits into the slide groove to detachably secure to the cylinder support member.

The stopper pin (1051) through the through hole regulates the movement of the syringe holder in the direction toward the tip.

This allows the syringe holder to be attached to the cylinder support member after the injector is set in place, making it easier to attach the syringe to the medical balloon pressurizer. In addition, the syringe can be quickly removed in the proximal direction of the shaft axis by removing the stopper pin. At this time, the plunger flange of the syringe is fixed to the shaft, so the plunger is pulled out of the syringe. In other words, it is possible to rapidly reduce the pressure applied to the medical balloon.

### Means of securing syringe and medical balloon pressurizer (FIG. 1 to FIG. 4, FIG. 13)

In the medical balloon pressurizer (120) of [112], the stopper pin (1051) has a stopper pin head (1052) whose bore diameter is larger than the through hole at the point where it protrudes on the side of the cylinder support member opposite the syringe.

This allows the stopper pin to be pulled out from the opposite side of the cylinder support member from the side to which the syringe is fixed by grasping the stopper pin head. The stopper pin can be easily removed.

### Means of securing syringe and medical balloon pressurizer (FIG. 1 to FIG. 4, FIG. 14)

In the medical balloon pressurizer (120) of [112], a stopper pin (1151) is fixed to a lever (1153) that is pivotably fixed by a hinge (1154) to the opposite side of the cylinder support member from the syringe and can be pulled out through a through hole (1145).

This allows the stopper pin to be quickly removed by rotating the lever to pull the stopper pin out of the through hole.

### Torque limiter setpoints (FIG. 1 to FIG. 4)

In the medical balloon pressurizer (120) of [104], the driver is configured to spin the handle and not transmit rotation to the shaft when a torque of 2 Nm or more is applied to the shaft.

This reduces pressurization so that no pressure is applied that would cause the balloon to rupture. Since there is no need to use conventional equipment to measure the internal pressure of the syringe, the overall cost of the medical balloon dilation system can be reduced.

### Invention of medical balloon pressurizer kit (FIG. 1 to FIG. 4)

In the medical balloon pressurizer (120) of [101], it is a medical balloon pressurizer kit (110) in which the injector is detachably attached to the syringe holder.

This allows the medical balloon to be dilated. The present invention is an invention of a medical balloon pressurizer kit, which consists of a medical balloon pressurizer with an injector attached.

### Means of securing shaft and plunger (FIG. 1 to FIG. 4, FIG. 15)

In the medical balloon pressurizer kit (110) of [116], the shaft and the plunger flange are connected by a connecting member.

This allows the balloon to be forcibly deflated in an emergency or other situation. More specifically, a driver is used to reverse the shaft, pulling the plunger back, to return the contrast agent injected into the balloon to the syringe, in order to deflate the balloon.

### Means of securing shaft and plunger (FIG. 1 to FIG. 3)

In the medical balloon pressurizer (120) of [117], the shaft has a through hole (231) near the tip, one end of the connecting pin (232) that passes through the through hole is bent to engage with the plunger flange, and the other end of the connecting pin is detachably fixed to the connecting pin fixing member (233) so that it cannot be pulled out of the through hole.

The plunger flange of the injector can be detachably secured to the tip of the shaft by the connecting pin and the connecting pin fixing member.

This allows the axial back and forth movement of the shaft to be transmitted to the plunger.

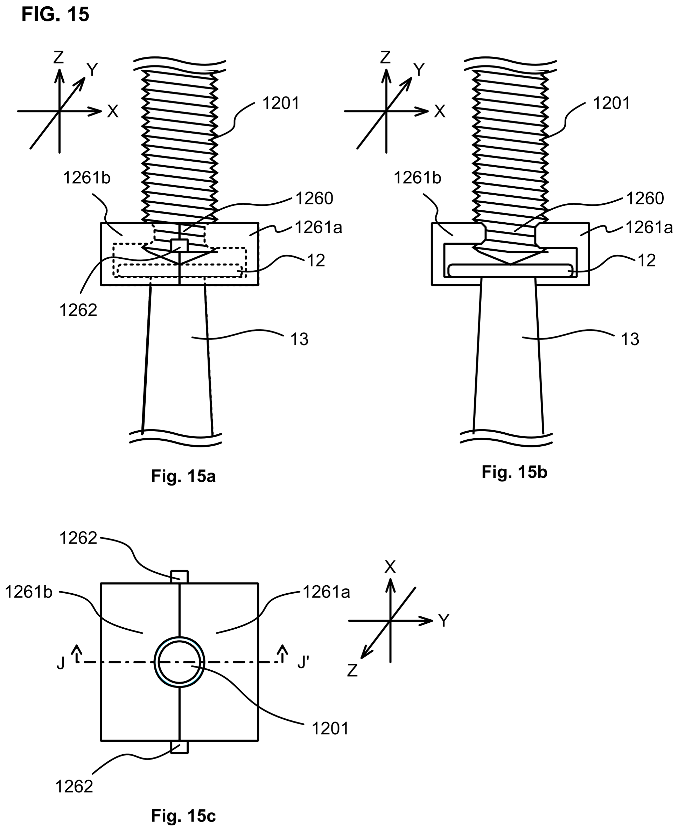

### Means of securing shaft and plunger (FIG. 1, FIG. 15)

In the medical balloon pressurizer (120) of [117], the shaft has a tapered, necked shaft cut out portion (1260) near the tip.

A plunger flange fixing member (1261a, b) capable of pinching the shaft cut out portion and the plunger flange is provided.

This allows the axial back and forth movement of the shaft to be transmitted to the plunger without transmitting shaft rotation to the plunger.

### 2. Details of the embodiments

Details of the embodiments will be described in more detail.

In the following, Embodiments 1 to 11 will focus on the embodiments corresponding to [101] to [119] above, and the latter Embodiments 12 and later will focus on the embodiments corresponding to [1] to [18] above.

In Embodiments 1 to 11, the medical balloon pressurizer 120 is basically composed of a shaft 1, a cylinder 2, a cylinder support member 3, a syringe holder 8, and a driver 9. On the other hand, in the latter Embodiment 12 and later, the shaft 1 and cylinder 2 are considered as a motion conversion mechanism that converts the rotational force of the driver 9 into a linear motion that pushes or pulls a plunger 13 of the injector attached to the syringe holder 8. Accordingly, the cylinder support member 3 is renamed simply "support member 3". That is, the shaft 1 and the cylinder 2 in Embodiments 1 to 11 are an embodiment of the motion conversion mechanism, and the cylinder support member 3 is an embodiment of the support member 3. Although a cantilevered cylinder support member 3 is illustrated in FIGs. 1 to 15, an embodiment in which two same-shaped cylinder support members 3 are arranged symmetrically across the central axis to support the cylinder 2 and the syringe holder 8 from both sides may alternatively be used.

### [Embodiment 1]

FIG. 1 is an explanatory view schematically illustrating the entire medical balloon dilation system 100 used to dilate a medical balloon 24 using a medical balloon pressurizer 120, which is the present invention. That is, FIG. 1 is an explanatory view illustrating an example configuration of a medical balloon dilation system 100 for dilating a medical balloon 24 using a medical balloon pressurizer kit 110 according to a first embodiment of the present invention.

The medical balloon pressurizer kit 110, in which an injector 11 is detachably secured to the medical balloon pressurizer 120, is connected to the medical balloon 24 via a medical tube 21 that is connected to a hub 16 of the injector, a Y-shaped connector 22, and a catheter shaft 23. A contrast agent injected into the medical balloon pressurizer kit 110 in advance is pressurized into the balloon that has been inserted into an affected area by manipulating the medical balloon pressurizer kit 110.

The medical balloon pressurizer kit 110 consists of the injector 11 fixed to the medical balloon pressurizer 120. This injector 11 can be a general-purpose, inexpensive injector.

The medical balloon pressurizer 120 consists of a shaft 1, a cylinder 2, a cylinder support member 3, a cylinder holder 8, and a driver 9.

The driver 9 equipped with a handle 91 and a socket 92 is secured to the shaft 1 by the socket 92. When the handle 91 is rotated, the socket 92 transmits the rotation to the shaft 1, causing the shaft 1 to rotate. The shaft 1 has external threads while the cylinder 2 has internal threads to engage with the shaft 1. Because the shaft 1 engages with the internal threads of the cylinder 2 and passes through the cylinder 2, rotating the shaft 1 moves the shaft 1 back and forth in the axial direction of the shaft 1 relative to the cylinder 2. Although the handle 91 and the shaft 1 are secured via the socket 92 in FIG. 1, the handle 91 and the shaft 1 may be secured directly without using the socket 92.

The cylinder support member 3 supports the cylinder 2 and a syringe holder 8. The syringe holder 8 can detachably secure a syringe 15 of the injector 11 by means of a syringe fixing section 7 and a finger flange fixing section 6. The syringe holder 8 may be formed integrally with the cylinder support member 3.

While the end of the shaft 1 opposite the socket 92 is connected by a connecting member 4 to a plunger flange 12 provided on the end of the plunger 13 of the injector 11 in FIG. 1, the connecting member 4 is not necessarily an essential component in the medical balloon pressurizer 120. Rotating the driver 9 moves the shaft 1 to the proximal side, which in turn pushes the plunger flange 12 and the plunger 13 into the syringe 15, thereby injecting the contrast agent into the medical balloon 24 via the medical tube 21, the Y-shaped connector 22, and the catheter shaft 23. This dilates the medical balloon 24. On the other hand, when the driver 9 is reversed to return the shaft 1 to the distal side, the tension and external pressure of the medical balloon 24 pushes back the injected contrast agent, which in turn pushes the plunger 13 back from the syringe 15 and moves it distally while in contact with the tip of the shaft 1. If the shaft 1 and the plunger flange 12 are connected by a connecting member, this connecting member 4 transmits the axial forward/backward movement of the shaft 1 to the plunger flange 12, so that the axial forward/backward movement of the shaft 1 is the movement of the plunger 13 of the injector 11. That is, by rotating the handle 9, not only when the contrast agent is dispensed from the injector 11 and injected into the medical balloon 24, but also when the contrast agent is withdrawn from the medical balloon 24 to depressurize, the medical balloon 24 can be depressurized and deflated without depending on the tension of medical balloon 24 or external pressure.

The driver 9 has a torque limiting mechanism and will not transmit a load in excess of a predetermined torque. In other words, if the load to rotate the shaft 1 exceeds a predetermined value when the handle 91 is rotated, the torque limiting mechanism of the driver 9 is activated and the handle 91 idles. Therefore, the shaft 1 does not rotate above a predetermined torque.

Because the medical balloon 24 is elastic, its internal pressure increases as it is injected with the contrast agent to expand. Thus, the injector 11 ejects the contrast agent at a stronger pressure as the medical balloon 24 expands. This means that the load to push the plunger 13 of the injector 11 is higher, and the load to move the shaft 1 to move this plunger 13 is also higher. This means that a higher torque is required to rotate the shaft 1 as the medical balloon 24 expands. In other words, the higher the internal pressure when the medical balloon 24 is injected with the contrast agent, the higher the load to rotate the shaft 1 correlates.

Because the torque limiting mechanism of the driver 9 prevents the shaft 1 from rotating above a predetermined torque, the medical balloon pressurizer kit 110 does not inject the contrast agent into the medical balloon 24 at pressures above a predetermined value. By setting the value set in this torque limiting mechanism below the value at which the medical balloon 24 could be ruptured, the medical balloon 24 will not be injected with the contrast agent at excessive pressure. This prevents the medical balloon 24 from rupturing if the handle 91 is rotated more than necessary. The torque limiting mechanism of the driver 9 may be provided in the socket 92 or inside the handle 91.

FIG. 2 is an explanatory view schematically illustrating front, side and cross-sectional structures to show an example of how the syringe 15 of the medical balloon pressurizer kit 110 is secured according to the first embodiment of the present invention. In FIG. 2, a medical balloon pressurizer body 230 without the driver 9 attached is shown in order to illustrate an example of how to secure the syringe 15.

A front view of the medical balloon pressurizer body 230 is shown in TOP VEIW. For simplicity of explanation, XYZ axes are set for convenience. Here, the x-axis corresponds to the left-right direction of the drawing, with x+ on the right and x- on the left. The Z-axis corresponds to the vertical direction of the drawing, with Z+ at the top and Z- at the bottom. The Y-axis corresponds to the direction from the back of the drawing to the front, with Y+ on the back and Y- on the front. A drawing of the medical balloon pressurizer body 230 in TOP VEIW viewed from the X+ direction is shown in SIDE VIEW. The A-A' section of SIDE VIEW is shown in the A-A' x-sectional view.

The medical balloon pressurizer body 230 consists of a shaft 201, a cylinder 202, a cylinder support member 203, and a syringe holder 208. The cylinder support 203 supports the cylinder 202 and the syringe holder 208. The shaft 201 passes through the cylinder 202 and can push the plunger flange 12 of the injector 11 in the shaft axis direction. The shaft 1 and the plunger flange 12 can be connected with the connecting member 4 as shown in FIG. 1. The syringe holder 208 consists of a syringe fixing section 207, a finger flange fixing section 206, and a syringe fixing section support member 205 in which these are provided. The syringe fixing section support member 205 and the cylinder support member 203 may be formed identically.

The syringe fixing section 207 is provided extending from both sides of the syringe fixing section support member 205 in the X-axis direction toward the direction in which the syringe 15 is fixed (Y- direction) in a shape along the outer surface of the syringe 15. The syringe fixing section 207 has the length that reaches a point where the syringe 15 narrows beyond the maximum width of thereof in the x-axis direction. In other words, the length of the syringe fixing section 207 is the length that the syringe 15 can be held so that it does not fall out.

The finger flange fixing section 206 consists of a shaft-side finger flange fixing section 206a and a syringe-side finger flange fixing section 206b. The shaft-side finger flange fixing section 206a and the syringe-side finger flange fixing section 206b are provided from both sides of the cylinder support member 203 in the X-axis direction toward the direction in which the syringe 15 is fixed (Y-direction). The finger flange 12 of the injector 11 is sandwiched between the shaft-side finger flange fixing section 206a and the syringe-side finger flange fixing section 206b, and is fixed so that it does not move in the shaft axis direction (Z-axis direction).

The shaft-side finger flange fixing section 206a and the syringe-side finger flange fixing section 206b have a length that reaches the point where it narrows beyond the maximum width of the syringe 15 in the X-axis direction as well as the syringe fixing section 207.

The shaft-side finger flange fixing section 206a, the syringe-side finger flange fixing section 206b, and the syringe 15 are formed of elastic resin or metal. Therefore, when mounting the injector 11 on the syringe holder 208, the injector 11 is pushed from the tip of the syringe fixing section 207 toward the cylinder support member 203 (Y- to Y+ direction), the syringe fixing section 207 is in turn pushed by the syringe outer surface of the injector 11, and then deforms to open. The injector 11 is pushed further, and when the open gap of the syringe fixing section 207 narrows beyond the maximum width, the syringe fixing section 207 and the syringe 15 are fitted and secured.

To remove the injector 11 from the syringe fixing section 207, the syringe 15 is pulled away from the syringe holder 208. Then the syringe fixing section 207 and the syringe-side finger flange fixing section 206b are pushed by the outer surface of the syringe 15, and the syringe fixing section 207 and the syringe-side finger flange fixing section 206b open, allowing the syringe 15 to be removed from the syringe holder 208. In this way, the injector 11 is detachably secured to the medical balloon pressurizer body 230. The elastic syringe fixing section 207 and syringe-side finger flange fixing section 206b can be formed by metal such as stainless steel or resin.

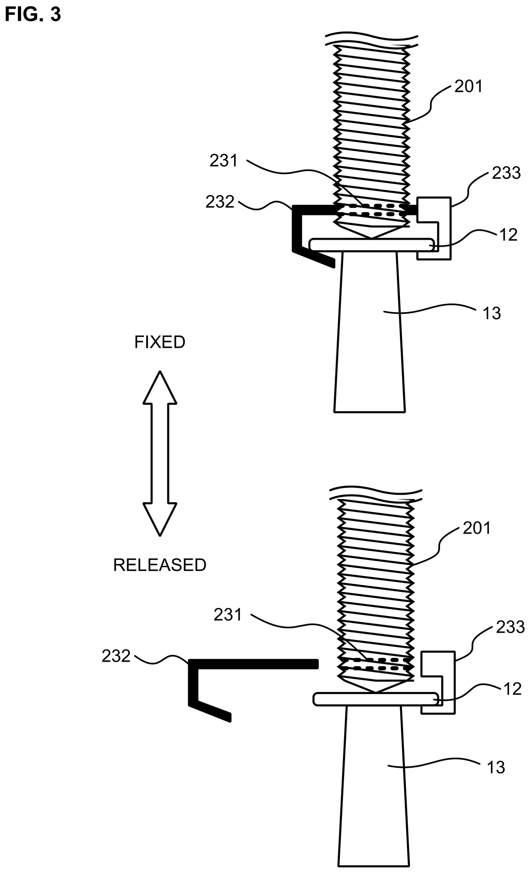

FIG. 3 is an explanatory view illustrating a connection structure of the shaft 201 and the plunger flange 12 of the injector 11 of the medical balloon pressurizer kit 110 according to the first embodiment of the present invention. The lower figure shows a connecting pin 232 and a connecting pin fixing member 233 before joining together, while the upper figure shows them joined together and the shaft 201 and the plunger flange 12 are secured to each other.

The shaft 201 has a through hole 231 near the tip. The connecting pin 232 is bent into a hook shape at one end and hangs on the plunger flange 12, while the other end passes through the through hole 231 of the shaft 201 and is fixed to the connecting pin fixing member 233. Regarding the fixing the connecting pin 232 and the connecting pin fixing member 233, for example, the connecting pin fixing member 233 has a hole into which the connecting pin 232 is inserted, and projections are provided near the tip of the connecting pin 232 and inside the hole; the engagement between the connecting pin 232 with the projections inside the hole of the connecting pin fixing member 233 enables connection and disconnection with a clicking feeling. The method of fixing the connecting pin 232 and the connecting pin fixing member 233 can be configured otherwise.

In addition, the connecting pin fixing member 233 hangs on the plunger flange 12. The plunger flange 12 is secured to the shaft 201 relative to the shaft axis direction (Z-axis direction) by the connecting pin 232 and the connecting pin fixing member 233.

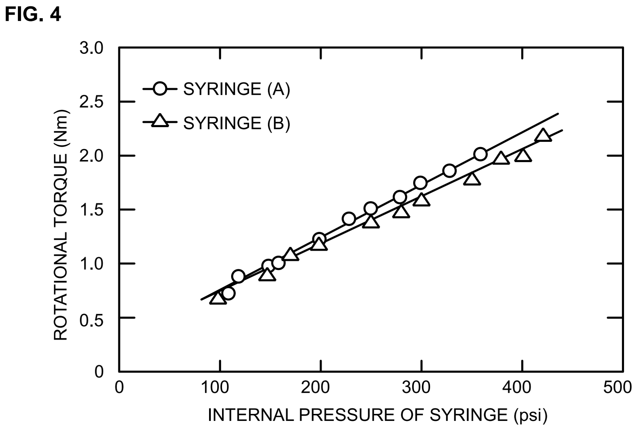

FIG. 4 is an explanatory view illustrating a correlation between the internal pressure of syringe 15 and the rotational torque of the shaft 1 of the medical balloon pressurizer kit (110, etc.) according to the present invention. The inventor of the present invention is a physician, and from his experience in performing many percutaneous kyphosis correction procedures, he has found a correlation between the internal pressure of a syringe 15 and values of rotational torque of the shaft 1. The following factors are related to the value of the internal pressure of the syringe 15 and the rotational torque of the shaft 1.

The first factor may be the length of the pitch of the shaft 1 screw; the greater the length of this pitch, the more contrast agent is fed in one rotation. This increases the internal pressure of the medical balloon 24 to a higher level. In other words, the larger the screw pitch, the higher the torque required to rotate the shaft 1.

The second factor may be the frictional force between the inner surface of the syringe 15 and the cylinder 2 and the shaft 1. The higher the frictional force, the higher the torque value required to rotate the shaft 1.

The third factor may be the size of the inner diameter of the syringe 15. If the inner diameter of the syringe 15 is larger, more contrast agent can be fed into the medical balloon 24 in the same amount of rotation of the shaft 1. Therefore, the larger the inner diameter of the syringe 15, the larger the torque value required to rotate the shaft 1.

The fourth factor may be the force with which the medical balloon 24 tries to deflate. In other words, the stronger the force with which the medical balloon 24 try to deflate, the higher the internal pressure of the medical balloon 24. Therefore, the stronger the force of the medical balloon 24 trying to deflate, the greater the torque value required to rotate the shaft 1.

The fifth factor may be the external pressure applied to the medical balloon 24. The higher the external pressure applied to the medical balloon 24, the higher the internal pressure of the medical balloon 24. Therefore, the higher the external pressure on the medical balloon 24, the higher the torque value required to rotate the shaft 1.

FIG. 4 is an explanatory view illustrating a correlation between the internal pressure of syringe 15 and the rotational torque of the shaft 1 of the medical balloon pressurizer kit 110 according to the present invention. Specifically, a special jig was made, the handle 91 of the driver 9 of the medical balloon pressurizer kit was set on the jig, and the end of the tube connected to the end of the hub 16 of the injector 11 was plugged to apply pressure for the measurement.

In FIG. 4, the horizontal axis shows the internal pressure of the syringe 15 (psi) and the vertical axis shows the rotational torque (Nm). Measurements were taken for two syringes A and B. Measurements shown as circles are for syringe (A) and measurements shown as triangles are for syringe (B). Both syringes had a relationship showing almost straight lines with its measurements increasing. Here, at an internal pressure of 150 psi, the rotational torque was about 1 Nm, at an internal pressure of 250 psi, the rotational torque was about 1.5 Nm, and at an internal pressure of 350 psi or higher, the rotational torque was over 2 Nm. Based on the results of this experiment, if the allowable upper limit of the balloon inner pressure is 400 psi, the system will not allow the balloon inner pressure to exceed 400 psi by setting the torque limit to 2 Nm or less.

### [Embodiment 2]

The medical balloon pressurizer 120 of the present invention can employ multiple embodiments and combinations thereof to facilitate the attachment and removal of the injector 11.

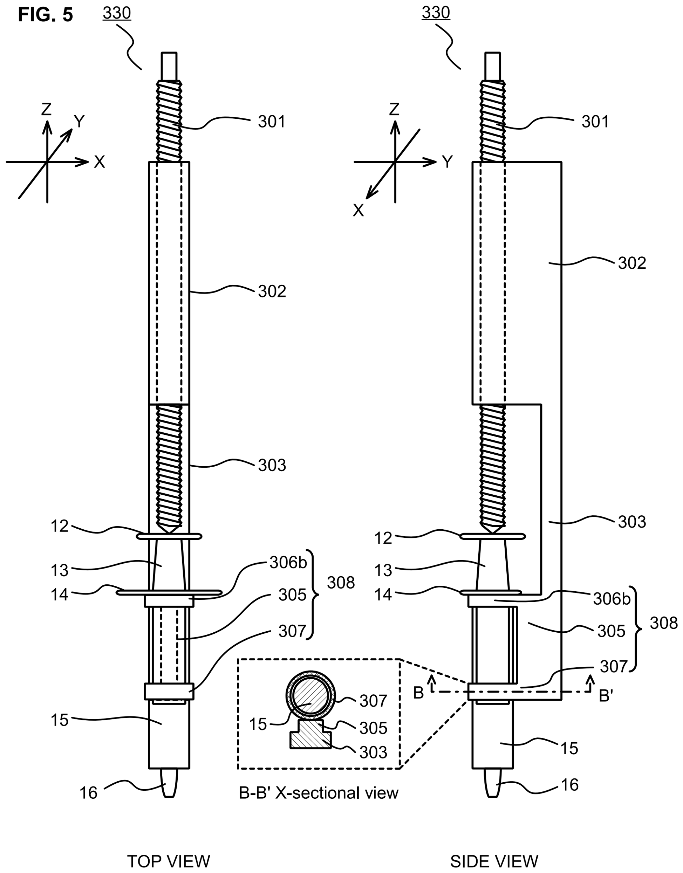

FIG. 5 is an explanatory view schematically illustrating front, side and cross-sectional structures to show an example of how a syringe 15 of a medical balloon pressurizer kit is secured according to a second embodiment of the present invention. In FIG. 5, the medical balloon pressurizer body 330 without a driver 9 attached is shown in order to illustrate an example of how to secure the syringe 15 as in FIG. 2.

The XYZ axis and TOP VEIW and SIDE VIEW shown in this figure are the same as in FIG. 2. The B-B' section of SIDE VIEW is shown in the B-B' x-sectional view. The configurations of a shaft 301, a cylinder 302, and a cylinder support member 303 are the same as those described in FIG. 2.

The syringe fixing section 307 and the shaft-side finger flange fixing member 306b are ring-shaped without any opening, and both are provided on the syringe fixing section support member 305. The inner diameter of these rings is sufficient to fit the syringe 15 inside. In the medical balloon pressurizer body 330 shown in FIG. 5, the syringe support member 305 and the cylinder support member 303 are formed integrally, but they may be formed separately.

In the present embodiment, when the injector 11 is attached to the medical balloon pressurizer body 330, the distance between the tip of the shaft 301 and the syringe-side finger flange fixing section 306b should be greater than the length of the injector 11. From the shaft 301 side of the syringe-side finger flange fixing section 306b, the hub 16 of the injector 11 is inserted into the ring of the syringe-side finger flange fixing section 306b, then into the ring of the syringe fixing section 307. The shaft 301 and the plunger flange 12 can be connected by a connecting member as shown in FIG. 3 or FIG. 15, and the injector 11 can be attached to the medical balloon pressurizer body 330.

When removing the injector 11 from the medical balloon pressurizer body 330, the shaft 301 is moved upward (Z+ direction). The injector 11 is then pulled out of the rings of the syringe fixing section 307 and the syringe-side finger flange fixing section 306b. The injector 11 is attached to and detached from the medical balloon pressurizer 320 in this manner.

In this embodiment, the syringe fixing section 307 and the shaft-side finger flange fixing member 306b are ring-shaped without any opening, so there is little risk that the injector 11 attached to the medical balloon pressurizer body 330 will unintentionally come off the medical balloon pressurizer body 330 due to impact or other factors.

### [Embodiment 3]

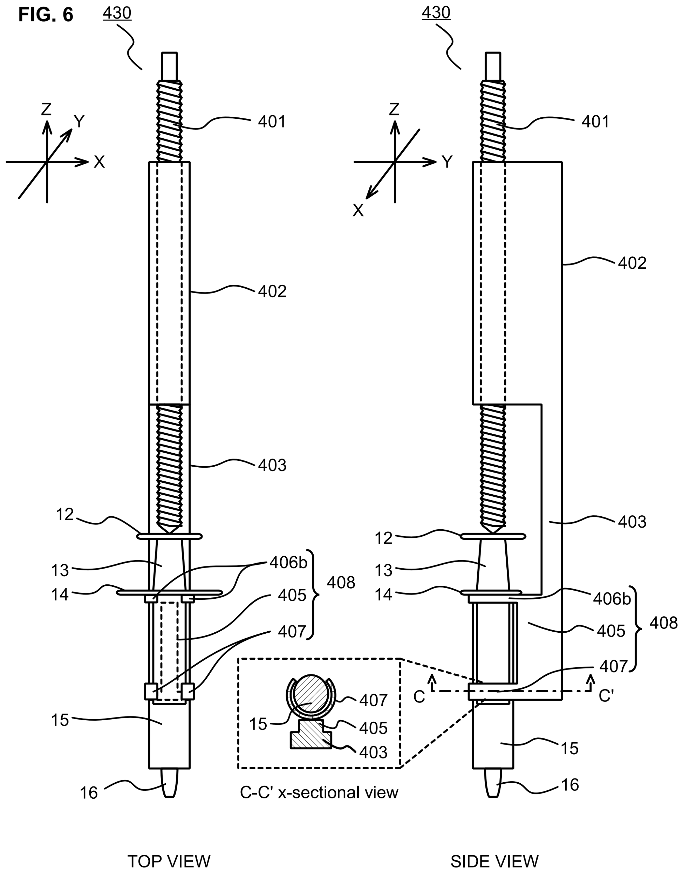

FIG. 6 is an explanatory view schematically illustrating front, side and cross-sectional structures to show an example of how a syringe 15 of a medical balloon pressurizer kit is secured according to a third embodiment of the present invention. In FIG. 6, a medical balloon pressurizer body 430 without a driver 9 attached is shown in order to illustrate an example of how to secure the syringe 15 as in FIG. 2.

The XYZ axis and TOP VEIW and SIDE VIEW shown in FIG. 6 are the same as in FIG. 2. The C-C' section of SIDE VIEW is shown in the C-C' x-sectional view. The configurations of a shaft 401, a cylinder 402, and a cylinder support member 403 are the same as those described in FIG. 2.

The medical balloon pressurizer body 430 shown in FIG. 6 differs from the medical balloon pressurizer body 330 shown in FIG. 5 in the shape of the syringe fixing section 407 and syringe-side finger flange fixing section 406b, but otherwise has the same configuration as the medical balloon pressurizer body 330 shown in FIG. 5. The shaft 401 and the plunger flange 12 can be connected by a connecting member as shown in FIG. 3 or FIG. 15, and the injector 11 can be attached to the medical balloon pressurizer body 430.

The syringe fixing section 407 extends in an arc shape from both sides of the syringe fixing section support member 405 in the direction in which the syringe 15 is mounted (Y-direction), and its tip has a gap sufficient to make the scale on the syringe 15 visible. The syringe-side finger flange fixing section 406b also has a gap in the same shape as the syringe fixing section 407.

In this embodiment, the syringe fixing section 407 and the syringe-side finger flange fixing section 406b have a gap where the scale on the syringe 15 can be seen, so a person operating the medical balloon pressurizer kit 110 equipped with the injector 11 can see the scale of the injector 11. This gap can be, for example, 1 cm large.

### [Embodiment 4]

FIG. 7 is an explanatory view schematically illustrating front, side and cross-sectional structures to show an example of how a syringe 15 of a medical balloon pressurizer kit is secured according to a fourth embodiment of the present invention. In FIG. 7, a medical balloon pressurizer body 530 without a driver 9 attached is shown in order to illustrate an example of how to secure the syringe 15 as in FIG. 2.

The XYZ axis and TOP VEIW and SIDE VIEW shown in FIG. 7 are the same as in FIG. 2. The D-D' section of SIDE VIEW is shown in the D-D' x-sectional view. The configurations of a shaft 501, a cylinder 502, and a cylinder support member 503 are the same as those described in FIG. 2. The shaft 501 and the plunger flange 12 can be connected by a connecting member as shown in FIG. 3 or FIG. 15, and the injector 11 can be attached to the medical balloon pressurizer body 530.

The syringe fixing section 507 and the syringe-side finger flange fixing section 506b are ring-shaped, as in the second embodiment. The inner diameter of these rings is sufficient to fit the syringe 15 inside. In the present embodiment, the syringe holder 508 and the cylinder support member 503 are separate. The syringe fixing section support member 505 has syringe holder protrusions 541 on the opposite side from the side on which the syringe fixing section 507 extends. The cross-section of the syringe holder protrusions 541 is tapered, with the width at the distance from the syringe holder 508 being greater than the width at the point closer to the syringe holder 508.

The cylinder support member 503 is provided with slide grooves 542 in a direction perpendicular to the shaft axis (X-axis direction). The cross-section of this slide groove 542 is shaped to fit with the syringe holder protrusion 541. In other words, the cross-section of the slide groove 542 is tapered, with the width of the opening narrower than the width of the bottom. In addition, at least one end of the slide groove 542 is open. The syringe holder 508 can be attached to the cylinder support member 503 by fitting and inserting the cylinder holder protrusion 541 from the open end of the slide groove 542 and sliding in the slide groove 542. The cross sections of the syringe holder protrusion 541 and the slide groove 542 are fitted together in a tapered shape, so the syringe holder 508 is fixed against the vertical (Y-) direction of the surface of the cylinder support member.

To set the injector 11 into the medical balloon pressurizer body 530, after the injector 11 is inserted into the shaft-side finger flange fixing section 506b and the syringe fixing section 507 and fixed to the syringe holder 508, the syringe holder protrusion 541 is fit into the slide groove 542 and slid. To remove the injector 11 from the medical balloon pressurizer body 530, the syringe holder 508 is removed from the slide groove 542, then the injector 11 is removed from the syringe holder. The injector 11 can be attached to and detached from the medical balloon pressurizer 520 in this manner.

There are two syringe holder protrusions 541 and two slide grooves 542 each In FIG. 7, but there can be one or more than two of each.

This structure of the present embodiment allows the syringe holder 508 to be attached to the cylinder support member 503 after the injector 11 is attached to the syringe holder 508, and vice versa for removal, so that the injector 11 can be easily attached to and removed from the medical balloon pressurizer.

### [Embodiment 5]

FIG. 8 is an explanatory view schematically illustrating front, side and cross-sectional structures to show an example of how a syringe 15 of a medical balloon pressurizer kit is secured according to a fifth embodiment of the present invention. In FIG. 8, a medical balloon pressurizer body 630 without a driver 9 attached is shown in order to illustrate an example of how to secure the syringe 15 as in FIG. 2.

The XYZ axes and TOP VEIW and SIDE VIEW shown in FIG. 8 are the same as those in FIG. 2. The E-E' section of SIDE VIEW is shown in the E-E' x-sectional view. The configurations of a shaft 601, a cylinder 602, and a cylinder support member 603 are the same as those described in FIG. 2. The shaft 601 and the plunger flange 12 can be connected by a connecting member as shown in FIG. 3 or FIG. 15, and an injector 11 can be attached to the medical balloon pressurizer body 630.

The configurations of a syringe holder 608, syringe holder protrusions 641, and slide grooves 642 in this embodiment are the same as in the fourth embodiment. Furthermore, the shapes of a syringe-side finger flange fixing section 606b and a syringe fixing section 607 in this embodiment are the same as those in the third embodiment, and a gap is provided where a scale of the injector 11 is visible. In other words, the present embodiment is a combination with the third embodiment.

The structure of this embodiment allows the syringe holder protrusions 641 and slide grooves 642 to easily attach and detach the injector 11, as in the fourth embodiment, and the scale of the attached injector 11 can be seen, as in the third embodiment.

### [Embodiment 6]

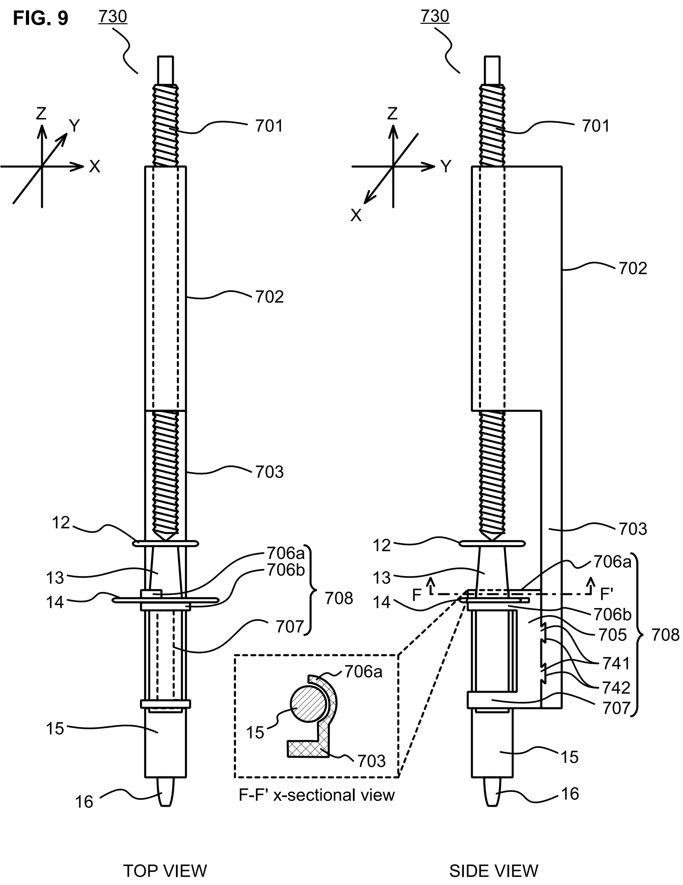

FIG. 9 is an explanatory view schematically illustrating front, side and cross-sectional structures to show an example of how a syringe 15 of a medical balloon pressurizer kit is secured according to a sixth embodiment of the present invention. In FIG. 9, a medical balloon pressurizer body 730 without a driver 9 attached is shown in order to illustrate an example of how to secure the syringe 15 as in FIG. 2.

The XYZ axes and TOP VEIW and SIDE VIEW shown in FIG. 9 are the same as those in FIG. 2. The F-F' section of SIDE VIEW is shown in the F-F' x-sectional view. The configurations of a shaft 701, a cylinder 702, and a cylinder support member 703 are the same as those described in FIG. 2. Furthermore, a syringe holder 708, syringe holder protrusions 741 and slide groove 742 are the same as those in the fourth embodiment. The shaft 701 and the plunger flange 12 can be connected by a connecting member as shown in FIG. 3 or FIG. 15, and the injector 11 can be attached to the medical balloon pressurizer body 730.

The medical balloon pressurizer body 730 of this embodiment has a syringe-side finger flange fixing section 706a on the cylinder support member 703. The syringe-side finger flange fixing section 706a extends from one end of the cylinder support member 703 in an arc shape to fix the syringe 15 so that it does not move in the shaft 701 direction (Z+ direction).

In other words, this embodiment has a syringe-side finger flange fixing section 706b on the medical balloon pressurizer body 530 of the fourth embodiment. This allows the injector 11 to be easily attached to and removed from the medical balloon pressurizer body 730, as in the fourth embodiment, and also restricts the movement of the attached injector 11 in the shaft 701 direction (Z+ direction) for stable fixation.

### [Embodiment 7]

FIG. 10 is an explanatory view schematically illustrating front, side and cross-sectional structures to show an example of how a syringe 15 of a medical balloon pressurizer kit is secured according to a seventh embodiment of the present invention. In FIG. 10, a medical balloon pressurizer body 830 without a driver 9 attached is shown in order to illustrate an example of how to secure the syringe 15 as in FIG. 2.

The XYZ axes and TOP VEIW and SIDE VIEW shown in FIG. 10 are the same as those in FIG. 2. The G-G' section of SIDE VIEW is shown in the G-G' x-sectional view. The configurations of a shaft 801, a cylinder 802, and a cylinder support member 803 are the same as those described in FIG. 2. The shaft 801 and the plunger flange 12 can be connected by a connecting member as shown in FIG. 3 or FIG. 15, and the injector 11 can be attached to the medical balloon pressurizer body 830.

A syringe fixing section 807 is provided at both ends of a syringe fixing section support member 805. The syringe fixing section 807 extends in an arc shape in the direction in which syringe 15 is mounted (Y- direction) and has a length that reaches a point where it narrows beyond the maximum width of the syringe 15 in the X-axis direction. Similar to the syringe fixing section 807, the syringe-side finger flange fixing section 806b is also provided at both ends of the syringe fixing section support member 805 and extends in an arc shape in the direction in which syringe 15 is mounted (Y- direction) and has a length that reaches a point where it narrows beyond the maximum width of the syringe 15 in the X-axis direction. That is, the shapes of the syringe-side finger flange fixing section 806b and the syringe fixing section 807 in FIG. 10 are the same as those of the syringe-side finger flange fixing section 206b and the syringe fixing section 207 in FIG. 2.

On the side of a syringe holder 808 opposite the syringe is provided a cylindrical syringe holder rotation axis 843. The syringe holder rotation axis 843 passes through a through hole 845 provided in the cylinder support member 803 and rotatably fixes the syringe holder 808 by a clamp 844.

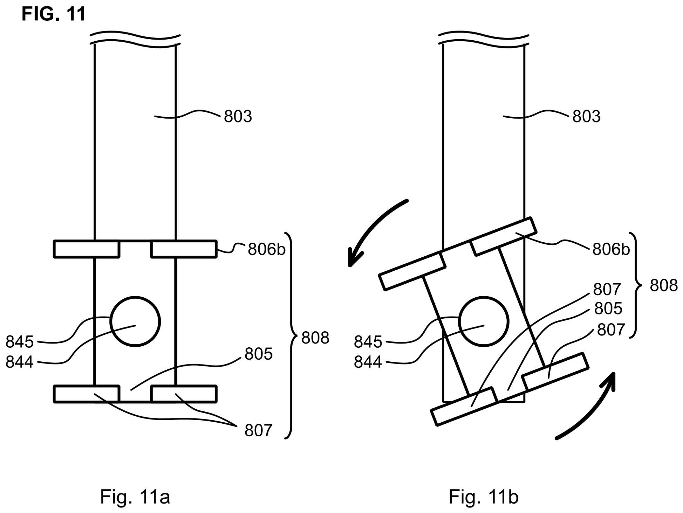

FIG. 11 is an explanatory view illustrating an example rotation of a syringe holder 808 of the medical balloon pressurizer kit according to the seventh embodiment of the present invention. FIG. 11a on the left side of FIG. 11 shows the position where the cylinder support member 803 and the long side of the syringe holder 808 become parallel to each other. FIG. 11b shows a counterclockwise rotation of the syringe holder 808 of FIG. 11a. When the cylinder holder 808 is in the position of FIG. 11a, it is difficult to mount the injector 11 on the syringe holder 808 because the injector 11 and the shaft 801 interfere with each other. However, as shown in FIG. 11b, by rotating the syringe holder 808, the injector 11 can be attached to the syringe holder 808 without interfering with the shaft 801. The same is true when removing the injector 11 from the syringe holder 808. This allows the injector 11 to be easily attached and detached in the present embodiment.

### [Embodiment 8]

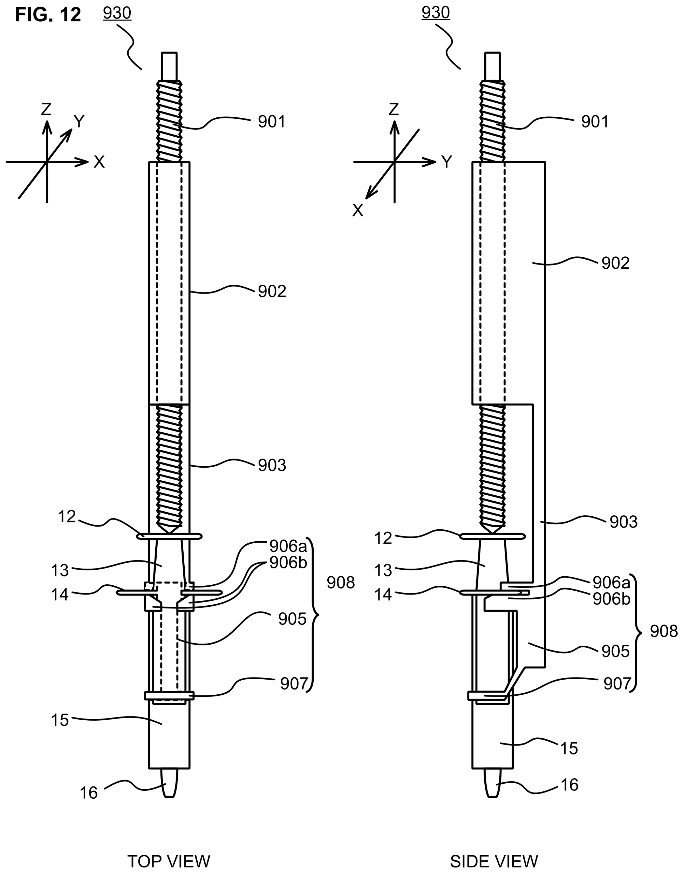

FIG. 12 is an explanatory view schematically illustrating front, side and cross-sectional structures to show an example of how a syringe 15 of a medical balloon pressurizer kit is secured according to an eighth embodiment of the present invention. In FIG. 12, a medical balloon pressurizer body 930 without a driver 9 attached is shown in order to illustrate an example of how to secure the syringe 15 as in FIG. 2. The shaft 901 and the plunger flange 12 can be connected by a connecting member as shown in FIG. 3 or FIG. 15, and the injector 11 can be attached to the medical balloon pressurizer body 930.

The XYZ axis and TOP VEIW and SIDE VIEW shown in FIG. 12 are the same as in FIG. 2. The configurations of a shaft 901, a cylinder 902, and a cylinder support member 903 are the same as those described in FIG. 2. Furthermore, a shaft-side finger flange fixing section 906a, a syringe-side finger flange fixing section 906b, and a syringe fixing section support member 905 have the same configurations as those in the first embodiment shown in FIG. 2. The shaft 901 and the plunger flange 12 can be connected by a connecting member as shown in FIG. 3 or FIG. 15, and the injector 11 can be attached to the medical balloon pressurizer body 930.

The syringe fixing section 907 extends from the end of the syringe fixing section support member 905 in a diagonal direction, which is the direction in which the syringe 15 is mounted (Y-direction) and away from the cylinder-side finger flange fixing section 906 (Z-direction). The syringe fixing section 907 has an arc shape that allows for the attachment of the syringe 15 inside. The part of the syringe fixing section 907 that contacts the syringe may be shaped to extend parallel to the syringe-side finger flange fixing section 907.

In this embodiment, when attaching the injector 11 to the medical balloon pressurizer body 930, the injector 11 is inserted into the syringe holder 908 from the upper direction (Y- and Z+ direction) of the injector setting face of the syringe holder 908, between the diagonally extending syringe fixing sections 907 and, as in the first embodiment, the syringe-side finger flange fixing section 906b is pushed open to attach the injector 11. To remove the injector 11 from the medical balloon pressurizer 920, the syringe holder 908 is moved upward (Y- and Z+ directions) on the surface where the injector 11 is set and remove it. In the first embodiment, it was necessary to push to open the syringe-side finger flange fixing section 206b and the syringe fixing section 207 when attaching and detaching the injector 11, but in this embodiment, only the syringe-side finger flange fixing section 906b needs to be pushed to open. Thus, the injector 11 can be attached and detached with less force than in the second embodiment.

### [Embodiment 9]

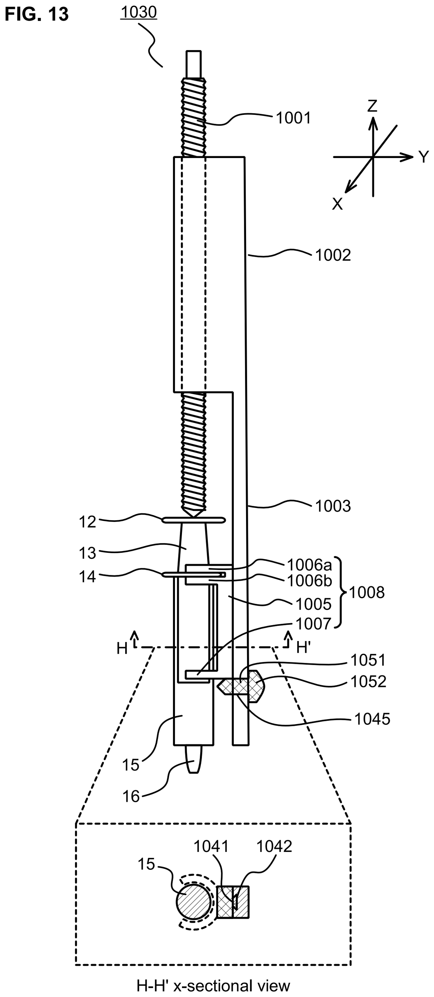

FIG. 13 is an explanatory view schematically illustrating side and cross-sectional structures to show an example of how a syringe 15 of a medical balloon pressurizer kit is secured according to a ninth embodiment of the present invention. In FIG. 13, a medical balloon pressurizer body 1030 without a driver 9 attached is shown in order to illustrate an example of how to secure the syringe 15 as in FIG. 2.

FIG. 13 shows SIDE VIEW perspective shown in FIG. 2. The H-H' cross section is shown in the H-H' x-sectional view. The configurations of a shaft 1001, a cylinder 1002, and a cylinder support member 1003 are the same as those described in FIG. 2. Furthermore, a syringe fixing section 1007, a syringe-side finger flange fixing section 1006b, and a shaft-side finger flange fixing section 1006a have the same structures as those in the first embodiment (FIG. 2). The shaft 1001 and the plunger flange 12 can be connected by a connecting member as shown in FIG. 3 or FIG. 15, and the injector 11 can be attached to the medical balloon pressurizer body 1030.

In this embodiment, a slide groove 1042 is provided on a syringe holder 1008 side of the cylinder support member 1003. The slide groove 1042 is provided in the axial direction (Z-axis direction) of the shaft 1 from the end opposite to the cylinder 1002 of the cylinder support member 1003, and is tapered with the width of the opening narrower than the width of the bottom of the cross section.

A syringe holder protrusion 1041 is provided on the opposite side of the syringe fixing section support member 1005 from the syringe 15. The cross-section of the syringe holder protrusion 1041 is tapered to engage with the cross-section of the slide groove 1042 described above. The syringe holder 1008 can be slid in the direction of the cylinder 1002 (Z+ direction) by fitting the syringe holder protrusion 1041 into the slide groove 1042 at the end of the cylinder support member 1003. The syringe holder 1008 is mounted by sliding it to where the syringe holder protrusion 1041, the end of the cylinder 1002 side (Z+ side) of the slide groove 1042, hits. The syringe holder 1008 is then restricted from moving in the direction opposite to the cylinder 1002 (Z- direction) of the cylinder support member 1003 by the stopper pin 1051 inserted into the through hole 1045 of the cylinder support member 1003 from the opposite side of the cylinder 1002. This secures the syringe holder 1008 in the front-back direction of the shaft axis (Z-axis direction). The syringe holder protrusion 1041 and the slide groove 1042 engage with each other in a tapered cross-sectional shape, so that movement in the direction of the syringe mounting surface (X-axis and Y-axis directions) is also restricted for fixing. The stopper pin 1051 can be inserted into or removed from the through hole 1045 by grasping the stopper pin head 1052 at the end.

In this embodiment, the injector 11 can be attached to and removed from the syringe holder 1008 with the syringe holder 1008 removed from the cylinder support member 1003, so attachment and removal of the injector 11 is easy. Furthermore, when the shaft 1001 and the plunger flange 12 of the injector 11 are connected by a connecting member, the stopper pin head 1052 is grasped to pull out the stopper pin 1051 and the injector 11 is moved in the cylinder support member 100 3 in the direction toward the tip (Z- direction) with the injector 11 set in the medical balloon pressurizer body 1030, then the plunger 13, which is connected to the shaft 1001 by a connecting member, is pulled out of the syringe 15 of the injector 11. This allows rapid depressurization of the internal pressure of the syringe 15 and of the medical balloon 24 connected to the medical balloon pressurizer body 1030. Even if, while pressurizing the medical balloon 24, the pressurization must be interrupted for some reason and the pressure must be reduced, the medical balloon pressurizer body 1030 of this embodiment can quickly reduce the pressure.

The syringe-side finger fixing portion 1006b and the syringe fixing section 1007 are arc-shaped with a gap In FIG. 13, but they can be ring-shaped as in the third embodiment.

### [Embodiment 10]

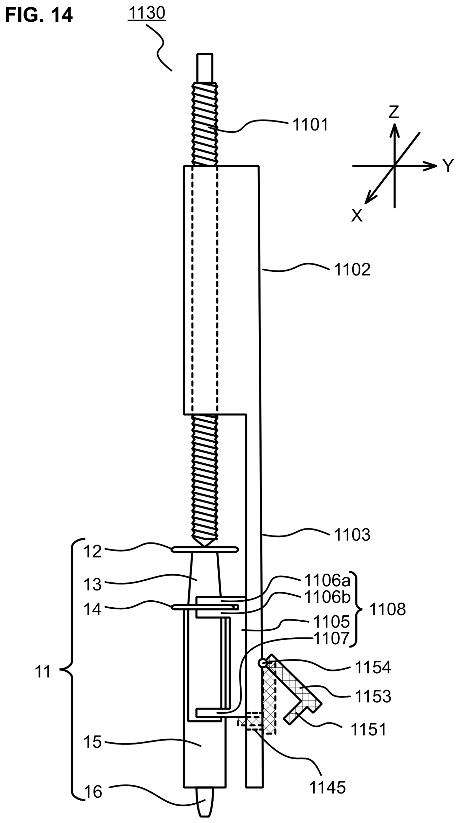

FIG. 14 is an explanatory view schematically illustrating the side structure of the medical balloon pressurizer body 1130 to show an example of how a syringe 15 of a medical balloon pressurizer kit is secured according to a 10th embodiment of the present invention. In FIG. 14, a medical balloon pressurizer body 1130 without a driver 9 attached is shown in order to illustrate an example of how to secure the syringe 15 as in FIG. 2.

FIG. 14 shows SIDE VIEW perspective shown in FIG. 2. The configurations of a shaft 1101, a cylinder 1102, a cylinder support member 1103, and a syringe holder 1108 are the same as those described in FIG. 2. Although the syringe holder protrusion and the slide groove are not shown in the figure, but their configurations are the same as those in the ninth embodiment. A stopper pin 1151 is provided in a lever 1153. The lever 1153 is rotatably attached to a cylinder support member 1103 by a hinge 1154. By rotating the lever 1153, the stopper pin 1151 can be inserted in and removed from the through hole 1145. The stopper pin 1151, inserted into the through hole 1145 by the lever 1153, protrudes on the opposite side of the cylinder support member 1103 from the lever to secure the syringe holder 1108 that fits into the slide groove (not shown). This secures the 1108 syringe holder in the shaft axis direction (Z-axis direction). The syringe holder 1108 of this embodiment is fixed to the slide groove (not shown) as in the medical balloon pressurizer 1020 of the ninth embodiment, so the syringe holder 1108 is also fixed to the direction in which the syringe is fixed (Y- direction).

The shaft 1101 and the plunger flange 12 can be connected by a connecting member as shown in FIG. 3 or FIG. 15, and the injector 11 can be attached to the medical balloon pressurizer body 1130.

In this embodiment, as in the ninth embodiment of the medical balloon pressurizer 1020, the syringe holder 1108 is secured to the cylinder support member 1103 by fitting the syringe holder protrusions to the slide groove (not shown), so the injector 11 can be easily attached and detached, as in the ninth example. Furthermore, by operating the lever 1153, the stopper pin 1151 can be quickly pulled out of the through hole 1145, which is a quicker and easier operation than pulling out the stopper pin 1051 of the ninth embodiment. In the ninth embodiment, the stopper pin 1051 that has not been set in the cylinder support member 1003 will roll away, and care must be taken to manage it. However, in the present embodiment, the stopper pin 1151 is fixed to the lever 1153, so there is no risk of the stopper pin 1151 rolling or being lost.

### [Embodiment 11]

FIG. 15 shows another embodiment of a connecting member 1233. FIG. 15a is a front view (from Y- to Y+ direction). FIG. 15c shows a view in a shaft 1201 direction (from Z+ to Z- direction). FIG. 15b is a J-J' cross section of FIG. 15.c.

In this embodiment, the connecting member consists of plunger flange fixing members 1261A and 1261B. As shown in FIG. 15b, the shaft 1201 has a tapered shaft cut out portion 1260 with a round-shaped section near the tip. Plunger connecting members 1261a and 1261b secure the shaft 1201 and the plunger 15 in the shaft axis direction (Z-axis direction) by fitting at their upper side (Z+ side) into the shaft cut out portion 1260 and abutting against the plunger flange 12 at the lower side (Z- side). Also, the plunger flange fixing members 1261a and 1261b are fitted with the shaft cut out portion 1260 with a round-shaped section, so that even when the shaft 1201 rotates, the plunger flange fixing members 1261a and 1261b and the plunger 12 do not rotate with the shaft 1201. The plunger flange fixing members 1261a and 1261b are secured to each other with a plunger flange fixing member clamp 1262.

This allows the back-and-forth (Z-axis direction) movement of the shaft 1201 to be transmitted to the plunger 13.

### [Embodiment 12]

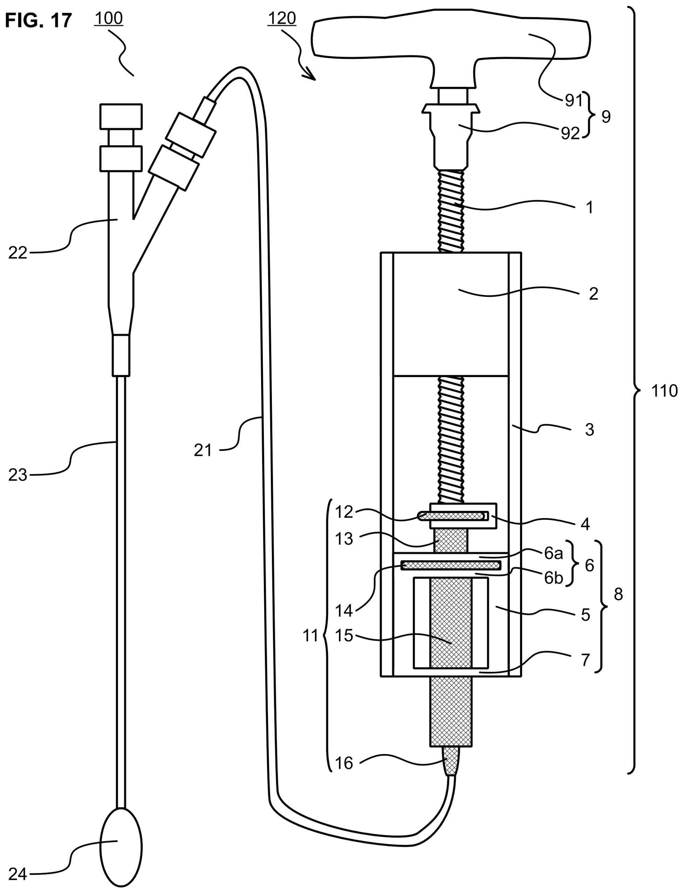

FIG. 17 is an explanatory view schematically illustrating the entire balloon dilation system 100 used to dilate a medical balloon 24 using a medical balloon pressurizer 120 according to a 12th embodiment of the present invention. As in Embodiment 1, which is described by citing FIG. 1, the medical balloon pressurizer 120 with an injector 11 attached is called a medical balloon pressurizer kit 110, to which the medical balloon 24 is connected through a medical tube 21, a Y-shaped connector 22, and a catheter shaft 23 connected to a hub 16 of the injector 11. The contrast agent injected into the medical balloon pressurizer kit 110 in advance is pressurized into the medical balloon 24 inserted into the affected area by operating the medical balloon pressurizer kit 110.

The medical balloon pressurizer 120 of Embodiment 12 has a syringe holder 8, a motion conversion mechanism (1, 2), a driver 9, and a support member 3, and is configured as follows.

The syringe holder 8 is attached to the proximal side of a support member 3 and can detachably support the injector 11 with a syringe 15 and a plunger 13.

The motion conversion mechanism is attached to the distal side of the support member 3 and can convert the rotational force of a driver 9 into a force that pushes the plunger 13 of the injector 11. In the example in FIG. 17, as in Embodiment 1 (FIG. 1) and other embodiments, the motion conversion mechanism consists of a shaft 1 with threads on its outer periphery and a cylinder 2 with threads on its inner periphery that engage with the threads.

The driver 9 has a handle 91 which provides a torque limiting function by idling when a torque to rotate the handle exceeds a predetermined value and not transmitting the torque to the motion conversion mechanism.

Since the medical balloon pressurizer and the injector are separate, only the injector needs to be replaced in a single surgery, and the medical balloon pressurizer does not need to be discarded and is thus reusable. Injectors are inexpensive because general-purpose ones can be used. This greatly reduces the economic burden on patients, since the price of materials to be disposed of after surgery can be less expensive.

The medical balloon pressurizer 120 of Embodiment 12 comprehensively includes Embodiments 1 to 11 described above. That is, the motion conversion mechanism is a mechanism that can convert the rotational motion of the driver 9 into a linear motion to push or pull the plunger 13 into or out of the syringe 15 of the injector 11.Although Embodiments 1 to 17, Embodiment 12, and Figures 1 to 3, 5 to 15, and 17 to 22 are described citing a simple example, which is composed of a shaft 1 and a cylinder 2,various implementations are possible, including those illustrated in Embodiments 18 and 19 below. The motion conversion mechanism consists of a threaded cylinder 32 and a ratchet structure 33 that engages with the threaded cylinder 32 in Embodiment 18, and consists of a threaded cylinder 2 and two threaded rods 42 that engage with the threads of the cylinder 2 in Embodiment 19.

Although a cantilevered cylinder support member 3 is illustrated in Embodiment 1 (FIG. 1), two cylinder support members 3 are arranged symmetrically across the central axis to support the cylinder 2 and the syringe holder 8 from both sides in this Embodiment 12. This ensures that the syringe holder 8 and the motion conversion mechanism (the shaft 1 and the cylinder 2) are firmly supported side by side on the same axis, reducing the influence of twisting and other effects caused by the rotation of the driver 9.

Other configurations and operations are the same as those in Embodiment 1, described citing FIG. 1.

### [Embodiment 13]

In the medical balloon pressurizer 120, it is suitable if the syringe holder 8 is configured so that it can be attached to and detached from the support member 3. This facilitates the attachment and removal of the injector 11 to and from the medical balloon pressurizer 120. This is because the syringe holder 8 can be removed from the support member 3, the injector 11 can be attached to the syringe holder 8, and then the syringe holder 8 with the injector 11 attached can be attached to the support member 3.

FIG. 18 is an explanatory view schematically illustrating an example configuration that allows the syringe holder 8 to be attached to and detached from the medical balloon pressurizer body 120 according to a 13th embodiment of the present invention. When the two support members 3 extend in the Z-axis direction and are arranged symmetrically with respect to the central axis in the Y-axis direction, the syringe holder protrusion 541 formed on the syringe fixing section support member 5 of the syringe holder 8 is fitted into the slide groove 542 formed on the support member 3, and can slide in the X-axis direction. The syringe fixing section support member 5 is formed integrally with the syringe-side finger flange fixing section 6b and the syringe fixing section 7, and is slid and attached to the support member 3 after the syringe section of the injector 11 is attached. The plunger flange 12 of the plunger 13 of the injector 11 is attached to the shaft 1, which is the linear motion side (pusher) of the motion conversion mechanism. These can be connected using the connecting member 4 as illustrated in FIG. 17, or employing the form described in Embodiment 11 (FIG. 15), or fitting by sliding the plunger flange 12 from the X-axis direction to prevent it from falling out in the Z-axis direction as illustrated in FIG. 18. The shaft-side finger flange fixing section 6a is then attached to the support member 3 by a sliding mechanism similar to that of the syringe fixing section support member 5.

### [Embodiment 14]

FIG. 19 is an explanatory view schematically illustrating another example configuration that allows an injector 11 to be attached to and detached from a medical balloon pressurizer body 120 according to a 14th embodiment of the present invention. In the medical balloon pressurizer 120, the syringe holder 8 is mounted, by a rotating mechanism (843 to 845) on the two beam-shaped support members 3, to be oriented in a direction in which the motion conversion mechanism is attached (+Z direction) and another direction (for example, +X direction). The inventive concept is the same as in Embodiment 7, which is described by citing FIGs. 10 and 11.

The syringe holder rotation axis 843 formed in the syringe fixing section support member 5 of the syringe holder 8 is passed through the through hole 845 in the support member 3 and fastened by the clamp 844. When the support members 3 are configured to be fixed to each other so that they cannot come off on both sides, the clamp 844 is not necessary, while the clamp 844 may function to fasten the two support members 3 so that they cannot come off on both sides.

The right side of FIG. 19 shows a state in which an injector 11 can be attached to and removed from a syringe holder 8. When the two support members 3 extend in the Z-axis direction and are arranged symmetrically with respect to the central axis in the Y-axis direction, by orienting the syringe holder 8 to the x-axis direction, the injector 11 can be attached to the syringe holder 8 without disturbing the motion conversion mechanism. As illustrated in FIG. 19, the shaft-side finger flange fixing section 6a is integrally formed with the support member 3 and does not rotate with the syringe holder 8. This configuration allows the finger flange 14 of the injector 11 to be held in contact with the shaft-side finger flange fixing section 6a when the injector 11 is rotated to its normal position as shown on the left side of FIG. 19 after being mounted, and the plunger flange 12 to be connected to the shaft 1 using the connecting member 4. Not only can the method of connecting the plunger flange 12 to the shaft 1, which is the pusher of the motion conversion mechanism, be changed to any other form, but the implementation of the motion conversion mechanism can also be changed as desired.

### [Embodiment 15]

FIG. 20 is an explanatory view schematically illustrating an example configuration that allows an injector 11 to be attached to and detached from a medical balloon pressurizer body 120 according to a 15th embodiment of the present invention. While the syringe holder 8 is detachable from the support member 3 in Embodiment 13, this embodiment is an example configuration in which the motion conversion mechanism is detachable from the support member 3.

In this embodiment, the motion conversion mechanism is detachably attached by being inserted and removed along a slide mechanism provided in the two support members 3. The two support members 3 have slide projections 38 on the side where they face each other. The slide projections 38 provided on the cylinder 2 on the side surface that contacts the support member 3 is fit into slide grooves 28, thereby supporting the cylinder 2 which is a part of the motion conversion mechanism. Two support members 3 extend in the Z-axis direction and face each other in the Y-axis direction, with the slide projections 38 and the slide grooves 28 formed in the X-axis direction to allow attachment and removal by sliding in the X-axis direction.

With the cylinder 2 and the shaft 1, which is the motion conversion mechanism, removed, the injector 11 is inserted into the syringe holder 8 from the direction in which the motion conversion mechanism should be installed (+Z direction), and then the cylinder 2 and the shaft 1, which is the motion conversion mechanism, are slid into the support member 3, followed by connecting the plunger flange 12 of the injector 11 and the shaft 1 by the connecting member 4. Thus, the injector 11 can be easily attached to and removed from the syringe holder 8.

The connection form between the plunger flange 12 and the shaft 1 may be changed to any other form, such as the form described in Embodiment 11 (FIG. 15), in addition to the form described above. FIG. 20 shows an example where the slide projections 38 are formed on the support members 3 and the slide grooves 28 are formed on the cylinder 2, which is the motion conversion mechanism, but the relationship between the projections and grooves can be reversed. The direction of the slide is described as the X-axis direction, but it does not necessarily have to be orthogonal to the direction in which the support members 3 extend (Z-axis direction), and may be changed to allow sliding from an angle for attachment and removal if operability is required. The motion conversion mechanism is described in the form consisting of the cylinder 2 and the shaft 1 as an example, but it may be changed to other forms of motion conversion mechanisms.

### [Embodiment 16]

FIG. 21 is an explanatory view schematically illustrating an example configuration that allows an injector 11 to be attached to and detached from a medical balloon pressurizer body according to a 16th embodiment of the present invention. While the motion conversion mechanism is detachable from the support member 3 in Embodiment 15, this embodiment is an example configuration in which the motion conversion mechanism can rotate on the support member 3.

In this embodiment, the motion conversion mechanism is mounted, by a rotating mechanism on the two support members, to be oriented in a direction in which the syringe holder 8 is attached and another direction. The two support members 3 have rotary holes 39 on the side where they face each other. A rotary shaft 29 provided on the cylinder 2 on the side surface that contacts the support member 3 is fit into the rotary holes 39, thereby supporting the cylinder 2 and the shaft 1 which is the motion conversion mechanism. Two support members 3 extend in the Z-axis direction and face each other in the Y-axis direction. The shaft 1, which constitutes the motion conversion mechanism, is tilted in a direction different from that in which the syringe holder 8 is attached (Z-axis direction) to create a gap on the upper side of the syringe holder 8 (+Z direction), through which the injector 11 can be inserted and attached to the syringe holder 8. After the injector 11 is attached, the motion conversion mechanism is returned to its original direction, i.e., to the Z-axis direction where the shaft 1 is the same as the support member 3, and the plunger flange 12 of the injector 11 and the shaft 1 are connected by the connecting member 4. Thus, the injector 11 can be easily attached to and removed from the syringe holder 8.

The connection form between the plunger flange 12 and the shaft 1 may be changed to any other form, such as the form described in Embodiment 11 (FIG. 15), in addition to the form described above. The mechanism with the rotary hole 39 and the rotary shaft 29 illustrated in FIG. 21 may be changed to other rotating mechanisms. The motion conversion mechanism is described in the form consisting of the cylinder 2 and the shaft 1 as an example, but it may be changed to other forms of motion conversion mechanisms.

### [Embodiment 17]

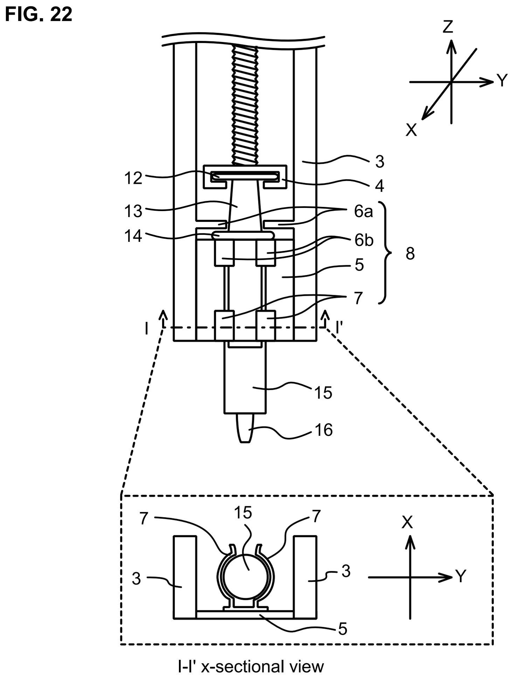

FIG. 22 is an explanatory view schematically illustrating an example configuration for securing an injector 11 to be attached to and detached from the medical balloon pressurizer body according to a 17th embodiment of the present invention. In addition to front view, an I-I' cross-sectional view is shown together in FIG. 22. Unlike in Embodiments 13 to 16, both the motion conversion mechanism and the syringe holder 8 are fixed to the support member 3.

Two support members 3 extend in the Z-axis direction and face each other in the Y-axis direction. A syringe fixing section support member 5 is fixed to bridge and connect those two support members 3. The syringe fixing section support member 5 is plate-shaped, fixed at one end (-X end) of the support member 3 in the thickness direction; a syringe-side finger flange fixing section 6b and a syringe fixing section 7, each formed by a plate spring clip are attached to an upper portion (+X direction) of the syringe fixing section support member 5. A plate spring clip is a mechanism in which elastic metal plates are bent to support a cylinder (syringe) from both sides, and when the plates on both sides are pushed apart for attachment, the curved plate spring wraps along the cylinder (syringe) to support the cylinder (syringe). The syringe-side finger flange fixing section 6b is located in contact with the finger flange 14 of the injector 11 to be mounted, and holds and fixes the finger flange 14 between the shaft-side finger flange fixing section 6a, which is provided protruding from the support member 3 on both sides with a gap sufficient to allow the plunger 13 to pass through. The injector 11 is inserted and attached from the +X direction by pushing open the plate spring clips that make up the syringe-side finger flange fixing section 6b and the syringe fixing section 7. At this time, the plunger 13 of the injector 11 passes through the above-described gap in the shaft-side finger flange fixing section 6a. After the injector 11 is attached, the motion conversion mechanism is returned to its original direction, that is, the direction in which the shaft 1 extends is returned to the Z-axis direction the same as the support member 3, and the plunger flange 12 of the injector 11 and the shaft 1 are connected by the connecting member 4. Thus, the injector 11 can be easily attached to and removed from the syringe holder 8.

The connection form between the plunger flange 12 and the shaft 1 may be changed to any other form, such as the form described in Embodiment 11 (FIG. 15), in addition to the form described above. The support mechanism for the injector 11 illustrated in FIG. 22 may be changed to other support mechanisms. For example, instead of a plate spring clip, a configuration including a non-elastic arc-shaped holder that curves along the syringe 15 of the injector 11, and a plate member that holds the syringe 15 from the +X direction after the injector 11 is attached for fitting and fastening may be employed.

### [Embodiment 18]

A medical balloon pressurizer 120 of the present invention is more suitable if a rapid depressurization mechanism is provided. In a balloon dilation system 100 of the present invention, the rotation of a driver 9 is converted by the motion conversion mechanism into a linear motion that pushes and pulls a plunger 13 of an injector 11, thereby increasing or decreasing the amount of contrast agent injected from the injector 11 and expanding and deflating a medical balloon 24. The ability to quickly withdraw the contrast agent from the injector 11 and deflate the medical balloon 24 may be required when the medical balloon 24 is excessively dilated due to unforeseen circumstances. It is more suitable to provide a rapid depressurization mechanism to allow the plunger 13 to be pulled out more quickly than the rotation of the driver 9.

FIG. 23 is an explanatory view schematically illustrating an example of a rapid depressurization mechanism in a medical balloon pressurizer body according to an 18th embodiment of the present invention.

The medical balloon pressurizer 120 of Embodiment 18 includes a driver 9, a motion conversion mechanism, a support member 3, and a syringe holder 8 attached to the support member 3. The motion conversion mechanism consists of a pusher 31 that is provided along the axis of rotation of the driver 9 and connected to the plunger flange 12 of the injector 11 that is mounted on the syringe holder 8, so that the motion conversion mechanism is configured to switch between a normal state and a rapidly depressurized state with the rotation of the driver 9, the normal state being a state in which the pushing and pulling of the plunger 13 into and out of the syringe 15 of the injector 11 is controlled by the change of distance between the syringe 13 and the pusher 31, and the rapidly depressurized state being a state in which the plunger 13 of the injector 11 can be withdrawn regardless of the rotation of the driver 9. This allows for rapid depressurization and deflation of the medical balloon 24 in unforeseen circumstances.

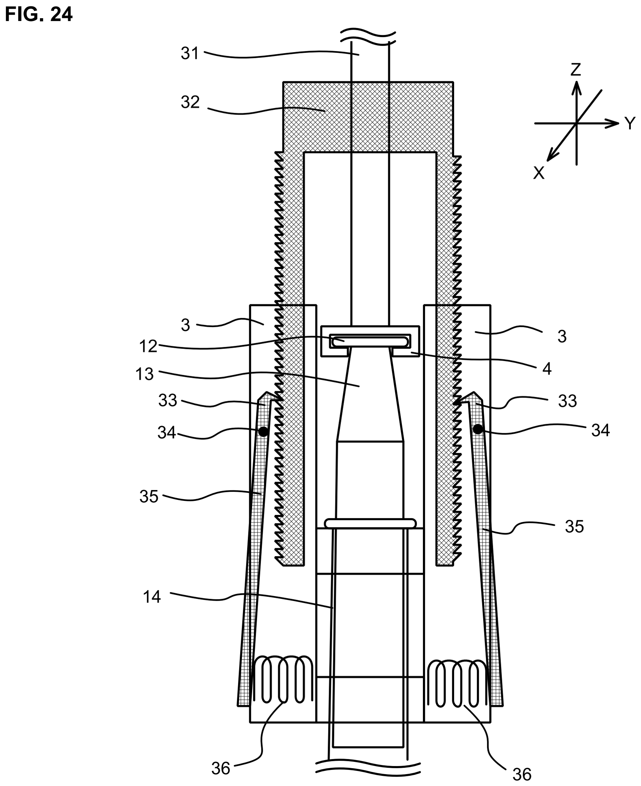

The motion conversion mechanism illustrated in FIG. 23 is the same mechanism as in FIG. 24 described below, but implementation thereof is arbitrary as long as it is configured to be able to switch between the normal and rapidly depressurized states described above.

FIG. 24 is an explanatory view schematically illustrating a first example of a rapid depressurization mechanism (coil spring) in the medical balloon pressurizer body according to the 18th embodiment of the present invention. The motion conversion mechanism in this embodiment consists of a pusher 31, a cylinder 32, a ratchet mechanism 33, a fulcrum 34, an operating lever 35, and an elastic body 36 (here, a coil spring is shown as an example). The pusher 31 is connected to and is rotated with a driver 9, and is also connected to the cylinder 32 to transmit its rotation to the cylinder 32.

The outer peripheral surface of the cylinder 32 has threads that engage with a ratchet mechanism 33 to convert the rotational motion of the cylinder 32 into linear motion of the connected pusher 31. The pusher 31 is connected at the tip (proximal end) to the plunger flange 12 of the injector 11 using a connecting member 4 or the like, and the above linear motion pushes or pulls the plunger 13 of the injector 11 against the syringe 15 fixed to the support member 3. This is the normal state in which the plunger 13 can be pushed in or pulled out as the driver 9 rotates.

The ratchet mechanism 33 is operated by an operating lever 35 across a fulcrum 34. Although the operating lever 35 is pushed away from the support member 3 by the coil spring 36 fixed to the support member 3, when the operating lever 35 is pushed to compress the coil spring 36 and bring it closer to the support member 3, the threads formed on the ratchet mechanism 33 are separated from the threads formed on the outer peripheral surface of the cylinder 32 and the screw engagement is released. This allows the cylinder 32 and the pusher 31 connected to it to move in a linear motion regardless of the rotation of the driver 9. This allows the plunger 13 connected to the pusher 31 to be rapidly withdrawn from the syringe 15. In other words, it is in a rapidly depressurized state. In this manner, the normal state and the rapidly depressurized state can be switched by operating the operating lever 35. In the rapidly depressurized state, the main purpose is to rapidly depressurize and deflate the medical balloon 24, but it is also possible to rapidly dilate the medical balloon 24 by pushing the plunger 13 into the syringe 15 in a linear motion without rotation of the driver 9.

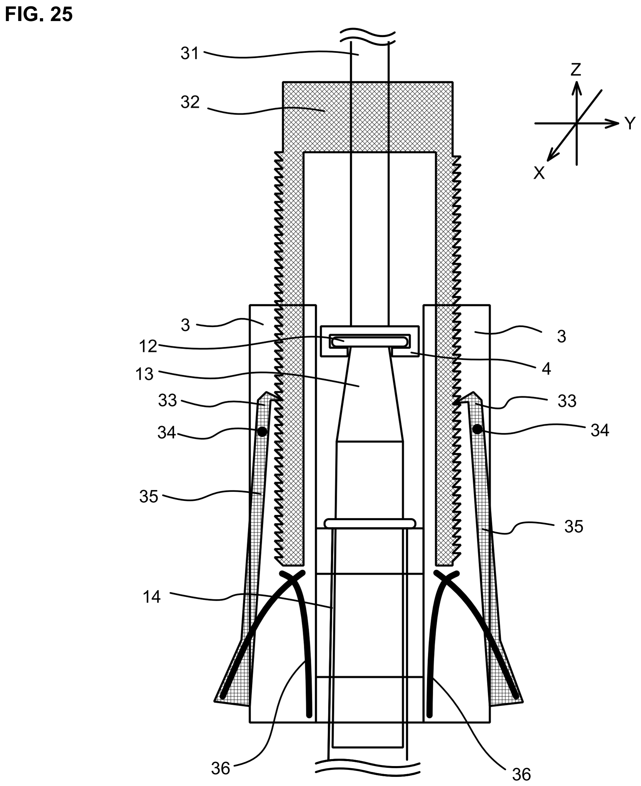

FIG. 25 is an explanatory view schematically illustrating a second example of a rapid depressurization mechanism (plate spring) in the medical balloon pressurizer body according to the 18th embodiment of the present invention. The elastic body fixed to the support member 3 that pushes the operating lever 35 away from the support member 3 has been replaced from the coil spring to a plate spring 36. An operating lever 35 protrudes greatly outward to accommodate the end of the plate spring 36 and is configured to aid in the pushing-in motion. Other configurations and operations are the same as in the example configuration described above, citing FIG. 24.

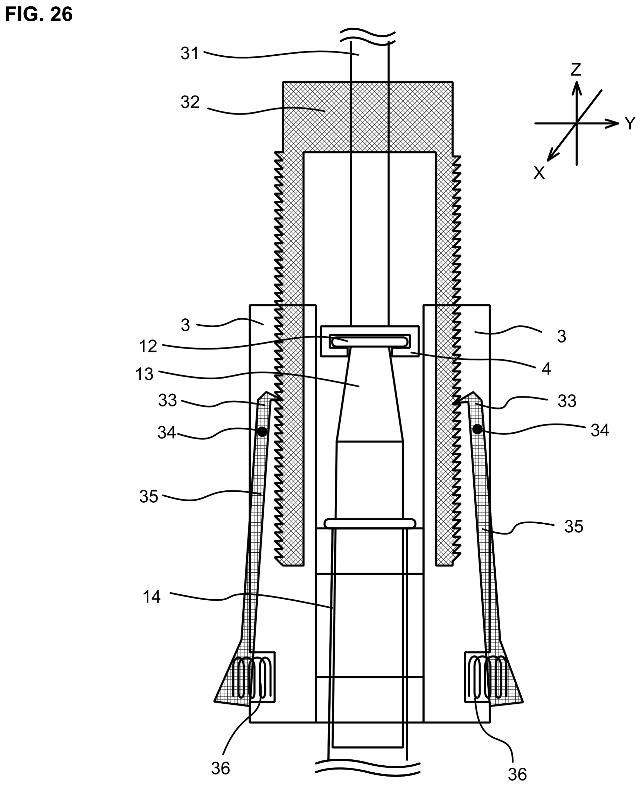

FIG. 26 is an explanatory view schematically illustrating a third example of a rapid depressurization mechanism (modification of the coil spring) in the medical balloon pressurizer body according to the 18th embodiment of the present invention. The elastic body fixed to the support member 3 that pushes the operating lever 35 away from the support member 3 is the coil spring 36 as in FIG. 24.However, the end on the support member 3 side is accommodated in a recess formed in the support member 3, and the other end is accommodated in a greatly protruding portion outward from the operating lever 35, which helps the push-in action as in FIG. 25. Other configurations and operations are the same as in the example configuration described above, citing FIG. 24.

The rapid depressurization mechanism of the embodiments illustrated in FIGs. 23 to 26 above is even more suitable if the cylinder 32 is configured so that it cannot be pulled out of the support member 3 even if the cylinder is pulled. The range of movement of the cylinder 32 is suitable to be the range where the plunger 13 cannot be pulled out of the syringe 15 in the injector to be connected.

### [Embodiment 19]

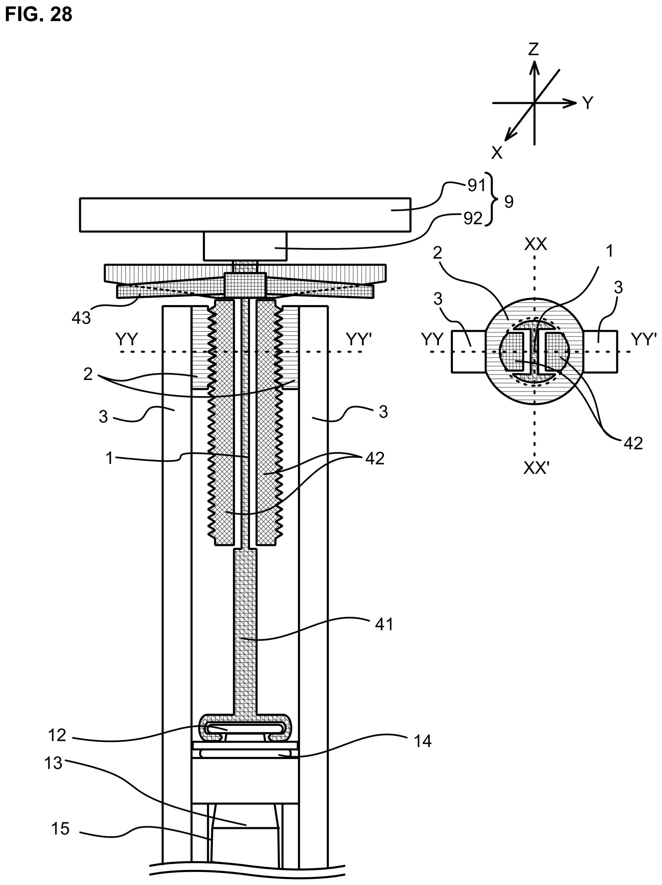

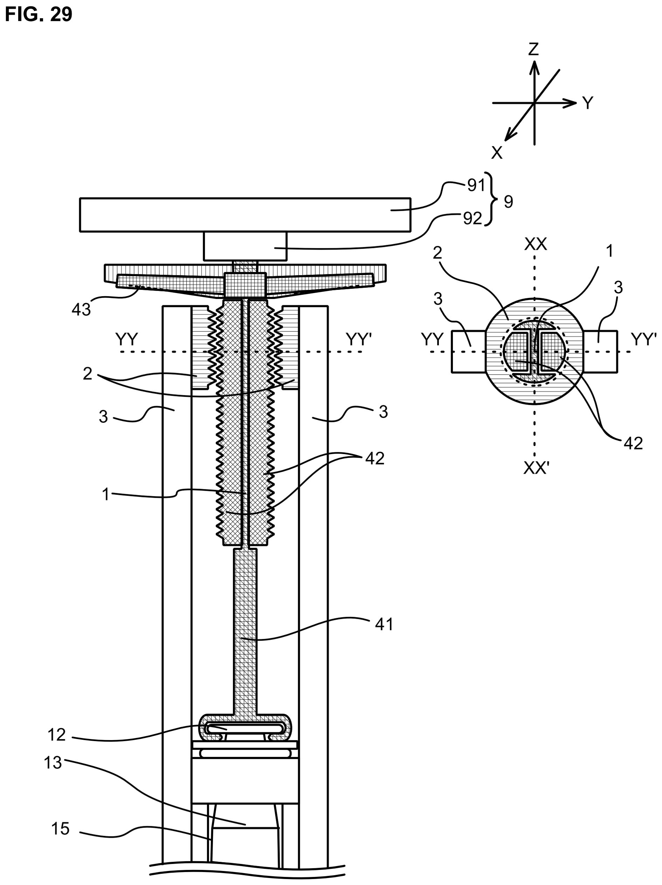

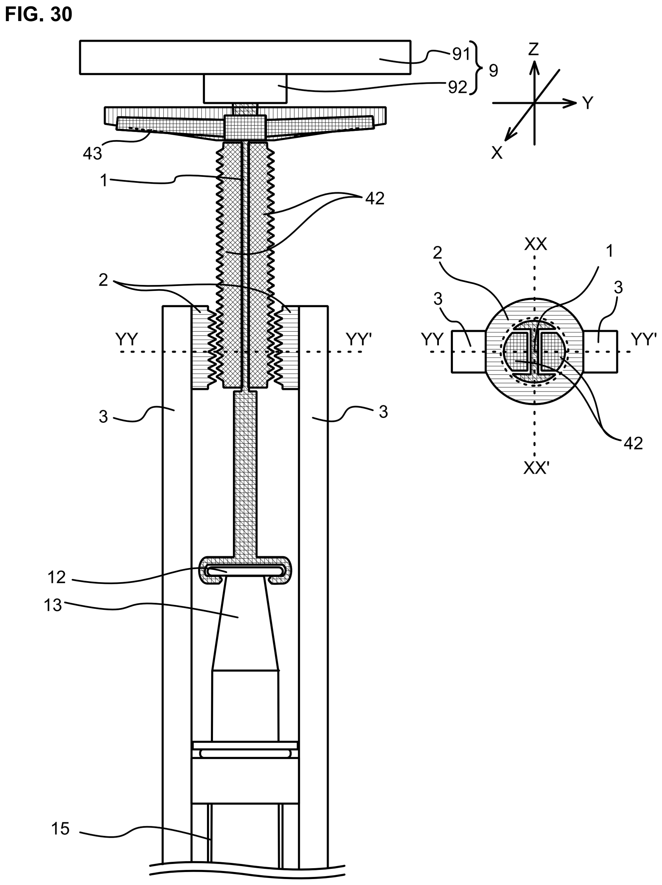

FIGs. 27 to 30 are explanatory views schematically illustrating an example of a rapid depressurization mechanism in a medical balloon pressurizer body according to a 19th embodiment of the present invention. FIG. 27 shows a state before the plunger is pushed in, FIG. 28 shows a state after the plunger is pushed in, FIG. 29 shows a state when the screw engagement is released, and FIG. 30 shows a state when the plunger is pulled out. On the right side of each, a YY-YY' cross section is shown. YY-YY' is the plane orthogonal to the Z-axis direction, which is the central axis of rotation of the driver 9 and the direction in which the support member 3 is located, and XX-XX' is the direction orthogonal to the YY-YY direction.

In the medical balloon pressurizer 120 of the present embodiment, the motion conversion mechanism includes a cylinder 2 that is fixed to the support member 3 and has threads on its inner peripheral surface, a shaft 1 that is inserted inside the cylinder 2 and rotates with the driver 9, with the pusher 41 connected on the central axis of rotation, and two rods 42 that have threads that engage with the threads of the cylinder 2. The shaft 1 has grooves formed on both sides of the central axis in a plane perpendicular to the central axis of rotation of the driver 9.

Each of the two rods 42 is placed in its normal state when it protrudes from its groove and the threads of rod 42 engage with the threads of the cylinder 2 (FIG. 27). The threads of the rod 42 engage with the threads of the cylinder 2, so when the driver 9 is rotated, the rod 42, the shaft 1, and the pusher 41 move in a linear motion to push or pull the plunger 13 into the syringe 15. FIG. 28 shows the plunger flange 12 pushed in deeply near the finger flange 14.

When the release lever 43 is operated, the rod 42 is pulled in the direction of the central axis and accommodated in the grooves formed in the shaft 1 to disengage the threads of the rod 42 and the threads of the cylinder 2 (FIG. 29). In this state, the driver 9, the shaft 1, the rod 42 and the pusher 41 can be moved linearly along the central axis without restriction by the threads of the cylinder 2 (i.e., a rapidly depressurized state). By pulling back the driver 9 while holding the release lever 43 in this rapidly depressurized state, the plunger 13 can be rapidly pulled back from being pushed into the syringe 15, rapidly deflating the connected medical balloon 24 (FIG. 30).

This allows the implementation of an easy-to-operate rapid depressurization mechanism in the motion conversion mechanism. The operation of the rod 42 can be performed on the driver side.

The rapid depressurization mechanism of the embodiment illustrated in FIGs. 27 to 30 is described with two rods 42 symmetrically provided, but the number of rods 42 can be one or more than two. When the number of rods 42 is one, more space is provided around the shaft 1 and the mechanism that moves the rod 42 by the release lever 43, thus reducing the overall size. When the number of rods is two or more, the rotation of the driver 9 can be stably transmitted to the shaft 1 by arranging them symmetrically about the central axis. In the rapid depressurization mechanism of the embodiment illustrated in FIGs. 27 to 30, the size of the connecting member of pusher 41 and the plunger flange 12 can be made larger than the inner diameter of the cylinder 2, so that the cylinder 2 cannot come off the support member 3 to which it is fixed. The range of movement of the pusher 41 is suitable to be the range where the plunger 13 cannot be pulled out of the syringe 15 in the injector to be connected.

### [Embodiment 20]

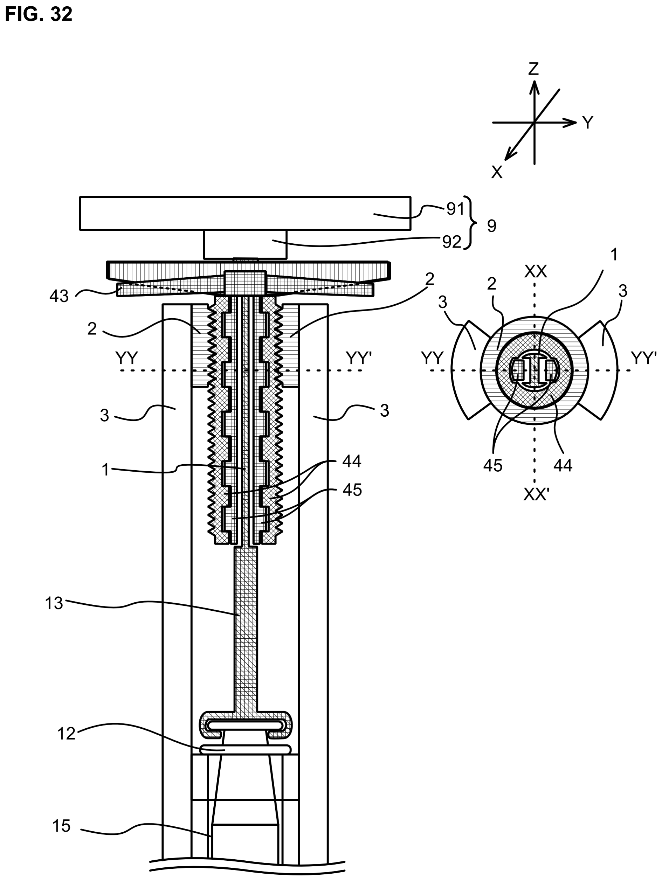

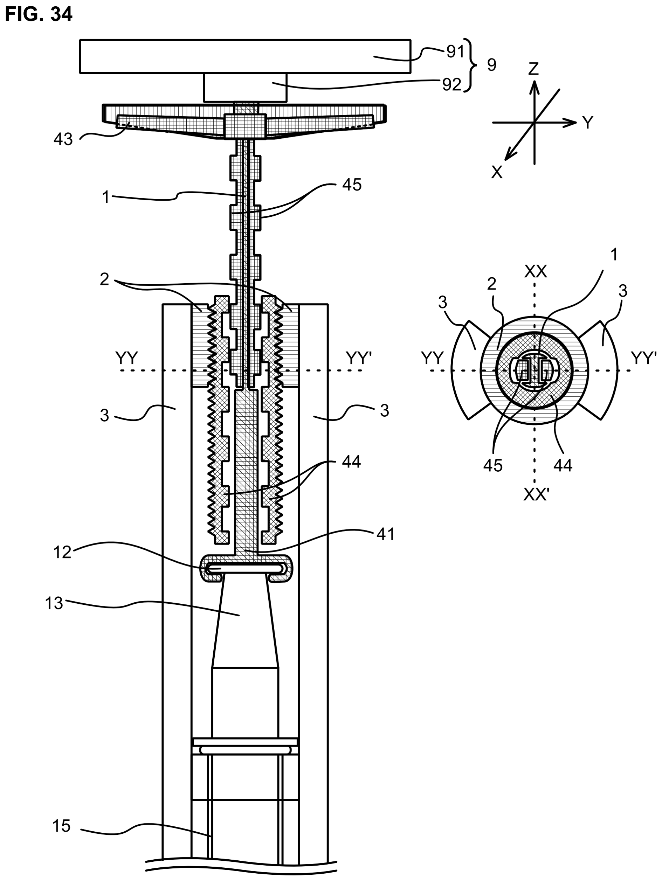

FIGs. 31 to 34 are explanatory views schematically illustrating an example of a rapid depressurization mechanism in a medical balloon pressurizer body according to a 20th embodiment of the present invention. A rapid depressurization mechanism by controlling presence or absence of connection between inner cylinder 44 and shaft, which engages with threads of cylinder 2 from shaft side 1 is configured. FIG. 31 shows a state before the plunger is pushed in, FIG. 32 shows a state after the plunger is pushed in, and FIG. 34 shows a state when the plunger is pulled out. On the right side of each, a YY-YY' cross section is shown. YY-YY' is the plane orthogonal to the Z-axis direction, which is the central axis of rotation of the driver 9 and the direction in which the support member 3 is located, and XX-XX' is the direction orthogonal to the YY-YY direction.

In the medical balloon pressurizer 120 according to the 20th embodiment of the present invention, the motion conversion mechanism includes a cylinder 2 that is fixed to the support member 3 and has threads on its inner peripheral surface, a shaft 1 that is inserted inside the cylinder 2 and rotates with the driver 9, with the pusher connected on the central axis of rotation, an inner cylinder 44 with threads that engage with the threads of the cylinder 2, and a rotation transmission rod 45. The shaft 1 has a groove in the plane perpendicular to the central axis, and the rotation transmission rod 45 protrudes from this groove so that protrusions formed on the rod 45 engages with recesses formed on an inner peripheral surface of the inner cylinder 44 to assume a normal state (FIG. 31). When the driver 9 is rotated in this state, the rotation of the rotation transmission rod 45, which rotates together with the driver 9 and the shaft 1, is transmitted directly to the inner cylinder 44, and the threads of the inner cylinder 44 and the cylinder 2 engage, converting the rotational motion of the driver 9 into a linear motion of the shaft 1; the plunger can be pushed in or pulled out as the driver 9 rotates. FIG. 32 shows the plunger flange 12 pushed in deeper near the funger fringe 14.

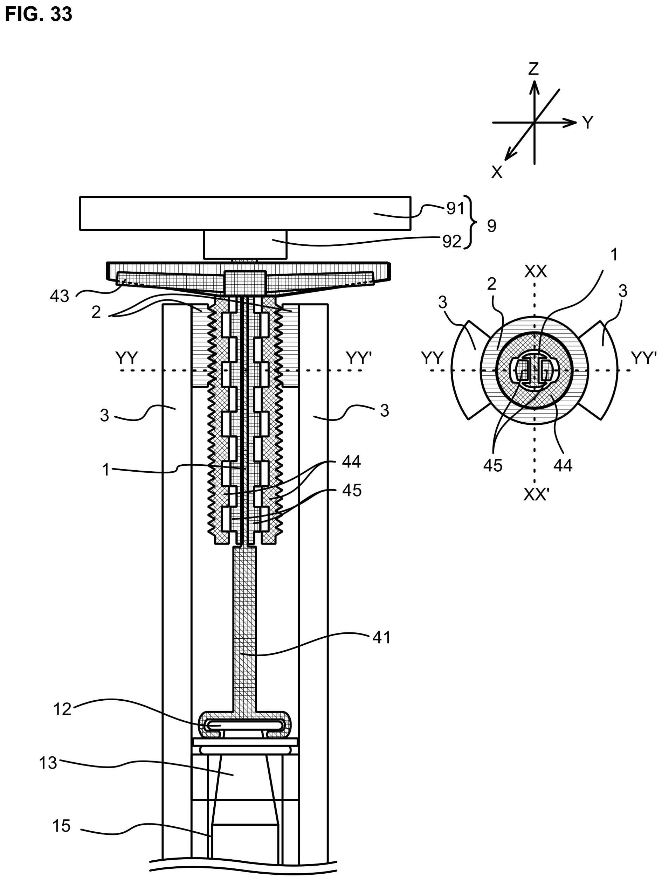

When the release lever 43 is operated, the rotation transmission rod 45 is pulled in the direction of the central axis and accommodated in a groove formed on the shaft 1, disconnecting the rotation transmission rod 45 from the inner cylinder 44 (FIG. 33). In this state, the driver 9, the rotation transmission rod 45 and the shaft 1 can be moved linearly along the central axis without restriction by the threads of the cylinder 2 (i.e., a rapidly depressurized state). By pulling back the driver 9 while holding the release lever 43 in this rapidly depressurized state, the plunger 13 can be rapidly pulled back from being pushed into the syringe 15, rapidly deflating the connected medical balloon 24 (FIG. 34).

This allows the implementation of an easy-to-operate rapid depressurization mechanism in the motion conversion mechanism. The operation of the rotation transmission rod 45, i.e., switching between the normal state and the rapidly depressurized state, can be performed on the driver 9 side.

The rapid depressurization mechanism of the embodiment illustrated in FIGs. 27 to 30 is described with two rotation transmission rods 45 symmetrically provided, but the number of rotation transmission rods 45 can be one or more than two. When the number of rotation transmission rods 45 is one, more space is provided around the shaft 1 and the mechanism that moves the rotation transmission rods 45 by the release lever 43, thus reducing the overall size. When the number of the rotation transmission rods 45 is two or more, the rotation of the driver 9 can be stably transmitted to the shaft 1 by arranging them symmetrically about the central axis. Although multiple protrusions and recesses formed on the rotation transmission rod 45 and the inner cylinder 44 are shown in the example, a single protrusion and a single recess (one on each for a rotation transmission rod 45) may be employed as well. Furthermore, by operating the release lever 43 or the like, it is possible to switch between connecting and disconnecting the portion that rotates with the driver 9 and the inner cylinder 44; if it is configured so that the inner cylinder 44 is left engaged with the cylinder 2 in the disconnected state and the shaft 1 can be pulled out, other mechanisms may be employed optionally. For example, the cylinder 2 illustrated in FIGs. 31 to 34 may be replaced by a columnar member with an arc-shaped base that engages with and supports the threads of the inner cylinder 44 from both sides or multiple locations.

In the rapid depressurization mechanism of the embodiment illustrated in FIGs. 31 to 34, the size of the connecting member of pusher 41 and the plunger flange 12 can be made larger than the inner diameter of the inner cylinder 44, so that the shaft 1 and the pusher 41 do not come off the support member 3 to which the inner cylinder 44 is fixed together with the cylinder 2. The range of movement of the pusher 41 is suitable to be the range where the plunger 13 cannot be pulled out of the syringe 14 in the injector to be connected.

While the present invention made by the present inventor has been described in detail with respect to the embodiments thereof, the present invention is not limited to these embodiments. Various changes may be made without departing from the spirit and scope of the present invention.

### Industrial Applicability

The present invention relates to a medical balloon pressurizer, which is particularly suitable for use in surgeries to dilate a spinal balloon to create a space to be filled with cement in fractured vertebrae or limb bones. The present invention is a reusable, medically economical medical balloon pressurization/depressurization device with a structure that allows the balloon dilation capacity to be checked with a syringe scale and is equipped with a mechanism to prevent a rise in the prescribed balloon internal pressure. The internal pressure measuring device may be omitted in cases where measurement of the internal pressure is not important when dilating a balloon in a blood vessel or in the heart, etc., in bones as well. The present invention can eliminate internal pressure measuring devices and is useful as an inexpensive medical balloon pressurizer.

### EXPLANATION OF SIGN

1 Shaft, pusher (plunger pusher)
2 Cylinder
3 Support member, cylinder support member
4 Connecting member
5 Syringe fixing section support member
6 Finger flange fixing section
6a Shaft-side finger flange fixing section
6b Syringe-side finger flange fixing section
7 Syringe fixing section
8 Syringe holder
9 Driver
11 Injector
12 Plunger flange
13 Plunger
14 Finger flange
15 Syringe
16 Hub
21 Medical tube
22 Y-shaped connector
23 Catheter shaft
24 Medical balloon
28 Slide groove
29 Rotation axis
31, 41 Pusher (plunger pusher)
32 Cylinder
33 Ratchet mechanism
34 Fulcrum
35 Operating lever
36 Elastic body (coil spring, plate spring)
38 Slide projection
39 Rotation hole
42 Rod
43 Release lever
44 Inner tube
45 Rotation transmission rod
91 Handle
92 Socket
100 Medical balloon dilation system
110 Medical balloon pressurizer kit
120 Medical balloon pressurizer
130 Medical balloon pressurizer body
231 Through hole
232 Connecting pin
233 Connecting pin fixing member
541 Syringe holder protrusions
542 Slide grooves
843 Syringe holder rotation axis (protrusion)
844 Clamp
845 Through hole
1051 Stopper pin
1052 Stopper pin head
1153 Lever
1154 Hinge
1260 Shaft cut out portion
1261a, 1261b Plunger flange fixing member
1262 Plunger flange fixing member clamp

## Claims

1. A medical balloon pressurizer comprising a syringe holder, a motion conversion mechanism, a driver, and a support member, wherein
the syringe holder can be attached to the proximal side of the support member to detachably support an injector provided with a syringe and a plunger;
the motion conversion mechanism can be attached to the distal side of the support member to convert a rotational force of the driver into a force that pushes the plunger of the injector; and
the driver comprises a handle which provides a torque limiting function by idling when a torque to rotate the handle exceeds a predetermined value and not transmitting the torque to the motion conversion mechanism.

2. The medical balloon pressurizer according to claim 1, wherein
the injector comprises a finger flange provided at a distal end of the syringe and a plunger flange provided at a distal end of the plunger;
the motion conversion mechanism comprises a rotary motion section that is connected to the driver and to which the torque is applied, and a linear motion section that contacts the plunger flange and pushes the plunger; and
the syringe holder comprises a syringe fixing section that detachably supports the syringe of the injector and a syringe-side finger flange fixing section that contacts and supports the proximal side of the finger flange of the injector.

3. The medical balloon pressurizer according to claim 2, wherein
the support member is formed as two beams, so that the syringe holder and the motion conversion mechanism are each supported by the two beam-shaped support members.

4. The medical balloon pressurizer according to claim 3, wherein
the motion conversion mechanism is detachably attached by being inserted and removed along a slide mechanism provided in the two beam-shaped support members.
This facilitates the attachment and removal of the injector to and from the syringe holder. This is because the injector can be attached by inserting the injector into the syringe holder from the direction in which the motion conversion mechanism should be attached while the motion conversion mechanism is removed, and then the motion conversion mechanism can be attached.

5. The medical balloon pressurizer according to claim 3, wherein
the motion conversion mechanism is mounted, by a rotating mechanism on the two beam-shaped support members, to be oriented in a direction in which the syringe holder is attached and another direction.
This facilitates the attachment and removal of the injector to and from the syringe holder. This is because, by directing the pusher extending from the motion conversion mechanism and pushing the plunger flange of the injector, to a direction different from that of the syringe holder, a space is created above the syringe holder, so the injector is inserted through that space into the syringe holder and attached; then the direction of the pusher is returned to its original position so that a member that pushes the plunger flange can be attached to the plunger flange of the injector attached to the syringe holder.

6. The medical balloon pressurizer according to claim 1, wherein
the syringe holder is detachably attached to the support member.

7. The medical balloon pressurizer according to claim 6, wherein
the support member is formed as two beams, so that the syringe holder and the motion conversion mechanism are each supported by the two beam-shaped support members.

8. The medical balloon pressurizer according to claim 1, wherein
the syringe holder is mounted, by a rotating mechanism on the two beam-shaped support members, to be oriented in a direction in which the motion conversion mechanism is attached and another direction.

9. The medical balloon pressurizer according to claim 8, wherein
the support member is formed as two beams, so that the syringe holder and the motion conversion mechanism are each supported by the two beam-shaped support members.

10. The medical balloon pressurizer according to claim 1, wherein
the motion conversion mechanism comprises a cylinder which has a female thread formed on its inner surface and a shaft which has a male thread formed that engages with the female thread on the inner surface of the cylinder and passes through the cylinder;
the driver is mounted so that the shaft can be rotated by the handle; and
the shaft rotates with the rotation of the handle and moves back and forth in the axial direction of the shaft through engagement with the female thread on the inner surface of the cylinder, and is able to move the plunger back and forth in the axial direction of the shaft via a plunger flange provided at a distal end of the plunger, which contacts the shaft at its tip.

11. The medical balloon pressurizer according to claim 1, wherein
the motion conversion mechanism comprises a pusher which is provided along an axis of rotation of the driver and is connected to the plunger flange of the injector,
so that the motion conversion mechanism can switch between a normal state and a rapidly depressurized state with the rotation of the driver, the normal state being a state in which the pushing and pulling of the plunger into and out of the syringe of the injector is controlled by the change of distance between the syringe and the pusher fixed to the syringe holder, and
the rapidly depressurized state being a state in which the plunger of the injector can be withdrawn regardless of the rotation of the driver.

12. The medical balloon pressurizer according to claim 11, wherein
the motion conversion mechanism comprises a cylinder that has threads on its outer peripheral surface and rotates with the driver, with the pusher connected on the central axis of rotation, and a ratchet structure that has threads that engage with the threads of the cylinder from the outside of the outer peripheral surface and is fixed to the syringe holder; and
the motion conversion mechanism is placed in the normal state by engaging the threads of the cylinder with the threads of the ratchet structure, and is placed in the rapidly depressurized state by a ratchet release by moving the threads of the ratchet structure outward from the outer peripheral surface of the cylinder to disengage them from the threads of the cylinder.

13. The medical balloon pressurizer according to claim 11, wherein
the motion conversion mechanism comprises a cylinder that is fixed to the support member and has threads on its inner peripheral surface, a shaft that is inserted inside the cylinder and rotates with the driver, with the pusher connected on the central axis of rotation, and one or more rods that have threads that engage with the threads of the cylinder;
the shaft has grooves in a plane perpendicular to the central axis; and
each of the one or two or more rods placed in the normal state when it protrudes from the groove and the threads of rod engages with the threads of the cylinder, and placed in the rapidly depressurized state when the rod is accommodated in the groove in the direction of the central axis and disengages the threads of the rod from the threads of the cylinder.

14. The medical balloon pressurizer according to claim 11, wherein
the motion conversion mechanism comprises a cylinder that is fixed to the support member and has threads on its inner peripheral surface, a shaft that is inserted inside the cylinder and rotates with the driver, with the pusher connected on the central axis of rotation, and a rotation transmission rod that has threads that engages with the threads of the cylinder;
the shaft has grooves in a plane perpendicular to the central axis; and
the rotary transmission rod is placed in the normal state when a protrusion protruding from the groove and formed on the rotation transmission rod engages with a recess formed on the inner circumference of the inner cylinder, and placed in the rapidly depressurized state when the engagement between the protrusion and the recess is released when the rotary transmission rod is accommodated in the groove in the direction of the central axis.

15. The medical balloon pressurizer according to claim 1, wherein
the predetermined value is 2 Nm.

16. A medical balloon pressurizer kit according to claim 1, wherein
the injector is detachably attached to the syringe holder.
